(19) Europäisches Patentamt
European Patent Office
Office européen des brevets

(11) **EP 3 586 123 B1**

(12) **EUROPEAN PATENT SPECIFICATION**

(45) Date of publication and mention
of the grant of the patent:
**08.03.2023 Bulletin 2023/10**

(21) Application number: **18709935.3**

(22) Date of filing: **14.02.2018**

(51) International Patent Classification (IPC):
**G01N 30/42** *(2006.01)*        **B01D 15/18** *(2006.01)*
**G01N 30/88** *(2006.01)*        **G01N 30/74** *(2006.01)*

(52) Cooperative Patent Classification (CPC):
**G01N 30/42; B01D 15/1807;** B01D 15/1864;
G01N 30/74; G01N 2030/8831; G01N 2030/889

(86) International application number:
**PCT/EP2018/053659**

(87) International publication number:
**WO 2018/153743 (30.08.2018 Gazette 2018/35)**

(54) **METHODS AND SYSTEMS FOR ADAPTING PATHLENGTH AND/OR WAVELENGTH OF A UV-ABSORBANCE CELL IN A CHROMATOGRAPHY SYSTEM**

VERFAHREN UND VORRICHTUNGEN ZUR ANPASSUNG DER OPTISCHEN WEGLÄNGE UND/ODER WELLENLÄNGE EINER ABSORPTIONSZELLE IN EINER CHROMATOGRAPHIE-VORRICHTUNG

MÉTHODES ET SYSTÈMES POUR ADAPTER LA LONGUEUR ET LA LONGUEUR D'ONDE D'UNE CELLULE DE DÉTECTION D'ABSORBANCE DANS UN SYSTÈME DE CHROMATOGRAPHIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.02.2017 US 201762461347 P**

(43) Date of publication of application:
**01.01.2020 Bulletin 2020/01**

(73) Proprietor: **Cytiva Sweden AB
751 84 Uppsala (SE)**

(72) Inventors:
• **BLOM, Hans
751 84 Uppsala (SE)**
• **BERG, Mikael
751 84 Uppsala (SE)**
• **EHRING, Hanno
751 84 Uppsala (SE)**
• **MATHIASSON, Linda
751 84 Uppsala (SE)**
• **SKOGLAR, Helena
751 84 Uppsala (SE)**
• **CHMIELOWSKI, Rebecka
Kenilworth, New Jersey 07033 (US)**

(74) Representative: **Bedford, Grant Richard
Global Life Sciences Solutions Operations UK Ltd
Amersham Place
Little Chalfont, Buckinghamshire HP7 9NA (GB)**

(56) References cited:
**WO-A1-2008/153472      WO-A1-2010/151214**

• POLLOCK JAMES ET AL: "Optimising the design and operation of semi-continuous affinity chromatography for clinical and commercial manufacture", JOURNAL OF CHROMATOGRAPHY A, ELSEVIER, AMSTERDAM, NL, vol. 1284, 25 January 2013 (2013-01-25), pages 17-27, XP029000701, ISSN: 0021-9673, DOI: 10.1016/J.CHROMA.2013.01.082
• RAHUL GODAWAT ET AL: "Periodic counter-current chromatography - design and operational considerations for integrated and continuous purification of proteins", BIOTECHNOLOGY JOURNAL, vol. 7, no. 12, 9 December 2012 (2012-12-09), pages 1496-1508, XP055144691, ISSN: 1860-6768, DOI: 10.1002/biot.201200068

- **VEENA WARIKOO ET AL: "Integrated continuous production of recombinant therapeutic proteins", BIOTECHNOLOGY AND BIOENGINEERING, vol. 109, no. 12, 6 December 2012 (2012-12-06), pages 3018-3029, XP055144587, ISSN: 0006-3592, DOI: 10.1002/bit.24584**

**Description**

TECHNICAL FIELD

The present invention relates to a method for determining operational status, e.g. binding capacities, of a chromatography column using UV detectors with selectable UV cell pathlength.

BACKGROUND

Binding capacity of a chromatography column for the solute is a very important factor in process chromatography. The binding capacity directly influences the productivity and cost of chromatography step. The binding capacity is defined either in terms of dynamic/breakthrough capacity or as the maximum binding capacity. The dynamic capacity depends on the conditions at which the solution flows through the column packed with chromatography medium, such as residence time defined as the ratio between column volume and feed flow rate. The maximum binding capacity represents a breakthrough capacity of the column if the residence time was at equilibrium. The initial breakthrough capacity is defined as the amount of binding solutes taken up by a column at the point when the solutes are first detected in the effluent. The breakthrough capacity can also be defined as a capacity at a given percentage of breakthrough, where the percentage represents the amount of binding solute present in the effluent from the column expressed in percent of the solute present in the feed. According to this definition the maximum binding capacity will be equal to breakthrough capacity at 100% of breakthrough, i.e., at the point where no more solute can bind to the column. Therefore, in order to determine maximum capacity, the breakthrough capacities are measured at different levels of breakthrough, where the levels are defined by levels of concentration of solutes measured in the effluent from the column during sample loading. Often these concentrations are determined by continuously monitoring a signal in a flowthrough detector placed in the effluent line. The plot of these concentrations (signal) against time (or volume or mass loaded) is called a breakthrough curve. Location of the breakthrough on a chromatogram and its shape is related to how much solute can bind on the column and how quickly all adsorption sites are saturated with the solute. It also shows how much more solute can be bound to the column at any given time. Breakthrough binding capacity for the solute in the presence of the impurities is one of the most critical parameters to optimize when developing a purification protocol. Because impurities more than often have similar light adsorbing properties as the solute determination of binding breakthrough capacities is a tedious and laborious work. In a typical experiment effluent from the column is collected in series of fraction, which are subsequently analyzed for the solute using high resolution techniques, such HPLC. Thus the determination of binding capacities for a chromatography column is rather complicated and in cases where the feed solution concentration is randomly varying during the feed application onto a chromatography column the true breakthrough capacities are intractable or extremely difficult to measure. The determination of binding capacity is very important if one wants to operate a column at the optimum process conditions. For instance, it can be shown that under certain conditions a maximum productivity of a capture chromatography step is obtained when the solute of interest reaches a certain value of its concentration in the column effluent, for instance a 10% of its initial concentration. If the breakthrough capacity is determined according to the method described above, it is impossible to terminate loading of the column at exact 10% breakthrough if either feed concentration or process conditions, including flow rate and/or chromatography media properties, vary with time in unpredictable manner.

Furthermore, determination of breakthrough capacities at different levels of breakthrough under varying process conditions is very important in the case of continuous chromatography.

In continuous chromatography, several identical columns are connected in an arrangement that allows columns to be operated in series and/or in parallel, depending on the method requirements. Thus, all columns can be run in principle simultaneously, but slightly shifted in method steps. The procedure can be repeated, so that each column is loaded, eluted, and regenerated several times in the process. Compared to 'conventional' chromatography, wherein a single chromatography cycle is based on several consecutive steps, such as loading, wash, elution and regeneration, in continuous chromatography based on multiple identical columns all these steps occur simultaneously but on different columns each. Continuous chromatography operation results in a better utilization of chromatography resin, reduced processing time and reduced buffer requirements, all of which benefits process economy. Continuous chromatography is sometimes denoted simulated moving bed (SMB) chromatography. Simulated moving bed chromatography is an example of periodic counter current process, because periodically all the chromatography columns comprising the system are simultaneously moved in the direction opposite to the sample flow. The apparent movement of the columns is realized by appropriate redirections of inlet and outlet stream to/from the columns.

Bishop et al ("Simulated Moving Bed technology in Biopharmaceutical Processing", Bischops, M. and Pennings, M., Recovery Biological Products XI, (2003) Banff, Alberta, Canada) discloses a continuous chromatography method based on simulated moving bed (SMB) technology, which has been successfully employed for the laboratory scale purification of IgG with a protein A affinity resin. Despite the fact that the multi-column and multi-zone continuous approach provided by SMB greatly increases process efficiency, SMB systems have not been utilized to date for cGMP biopharmaceutical production, mainly because of system complexity from both hardware and operational perspectives. The operational perspective is of particular interest as the continuous methods are more complex and require many operations (steps) to occur simultaneously at very precisely predefined points in time. In contrast to batch chromatography, implementation of safety factors to account for process variability is not recommended for continuous processes as by definitions they operate on the assumption of a steady state that can only be established if there is no variability in the input to the system.

In fact, simulated moving bed technology has been utilised for decades in various other fields. For example, US 3,291,726 (Universal Oil Products) described as early as 1966 a continuous simulated counter-current sorption process for the petrochemical industry.

Heeter et al (Heeter, G.A. and Liapis, A.I., J. Chrom A, 711 (1995)) have suggested, as an alternative to a typical four zone SMB system, a method based on a three column periodic counter-current chromatography (3C-PCC) principle. More recently, Lacki et al ("Protein A Counter-Current Chromatography for Continuous Antibody Purification", Lacki, K.M. and Bryntesson, L.M., ACS (2004) Anaheim, CA USA) described the use of such a 3C-PCC system for IgG adsorption to MabSelect™ affinity resin. This 3C-PCC method requires simpler hardware and easier operation than the typical four zone SMB system, directly reducing the cost associated with the capital equipment and the maintenance of the system.

Historically, essential factors for a reliable continuous process are: 1) the quality of the columns used, and more specifically the similarity or even identity between columns, 2) constant feed composition, and 3) hardware reliance, more specifically constant flow rate delivered by pumps. If the columns are not identical, the theoretical calculations typically used to design continuous chromatography process will not be correct, and it will become difficult to design an efficient and robust continuous chromatography process. The same argument applies if feed concentration and flow rates vary with time in an unexpected manner. Therefore, for scale-up considerations, having identical columns, reliable pumps in the system is essential. However, the packing of a column with a chromatography media is very complex in order to obtain repeatable results. Even small differences in the number of plates or other packing properties can have a huge effect on the end result. Furthermore, since capacities of chromatography resins typically change during resins lifetime/usage the process conditions chosen for a fresh resin will not be applicable for a resin that has been used several times. If also the feed solution concentration will vary it will be even more complicated to design an efficient continuous chromatography process that would operate at its optimum all the time. Normally, the composition of the feed material (sample) will vary over time based on the process for producing the sample. Thus, a detector used to determine the composition of the feed material into the chromatography process has to be able to monitor variations in feed composition, i.e. variations in the concentration of target compound in the sample. WO2010/151214 A1 (GE HEALTHCARE BIO SCIENCES, 29-12-2010) discloses a method for determining binding capacities of chromatography column, said method involving detecting feed signal representative of composition of feed material, detecting effluent signal representative of the composition of effluent and using feed and effluent signals.

SUMMARY

An object of the invention is to provide a reliable and dynamic method for determining and monitoring operational status of a chromatography column.

This is achieved by a method according to claim 1. Hereby the UV absorbance of the feed signal and the effluent signal can be used to continuously determine operational status of the chromatography column.

A further object of the invention is to provide a reliable and dynamic method for controlling a chromatography system.

This is achieved in a method according to claim 28. Hereby the chromatography system can be controlled dynamically based on real time measurements of the operational status of the chromatography column.

In one embodiment of the invention the chromatography system is a periodic counter current system.

Further suitable embodiments of the invention are defined in the dependent claims.

BRIEF DESCRIPTION OF THE DRAWINGS

Figure 1 shows schematically a chromatography system comprising one chromatography column and two detectors.

Figure 2 is a diagram showing the signals from the two detectors in Figure 1.

Figure 3 shows schematically a three column periodic counter current (3C-PCC) system comprising four detectors.

Figures 4 a, b, and c shows schematically three valves of Figure 3.

Figure 5 shows schematically a four column periodic counter current (4C-PCC) system according to the invention.

Figure 6 is a diagram showing the signals detected from the five detectors in the 4C-PCC system shown in Figure 5.

Figure 7 illustrates a schematic of a typical flow cell absorption monitor system.

Figures 8A to 8C show a schematic optical flow cell detector.

Figures 9A and 9B show a schematic view of a PCC system

Figure 10 shows a schematic view of dynamic control function in a PCC system

Figures 11A and 11B show antibody breakthrough for 0.5 mm versus 2 mm UV cell pathlength

Figures 12A-12D show UV absorbance as a function of UV cell pathlength

Figures 13A-13D show chromatography of protein loading

Figures 14A-14D show yield data and quality data for the FNVIP pools

Figure 15 illustrates UV 300nm absorbance as a function of UV pathlength

DETAILED DESCRIPTION

To circumvent the difficulties discussed in relation to prior art a real time control algorithm based on feedback like control principle is provided by the present invention. Accordingly, methods for assessing state of different columns at any given moment of the process are of particular interest. For instance, knowing of an operational status, e.g. binding capacity, of a chromatography column at a particular level of breakthrough would allow one to assess if the column can still bind solutes and how much solute still can be bound before the column reaches full saturation. Similarly, it is of paramount interest from the process yield perspective to know if the initial breakthrough capacity has been reached, as at this point the solute will be found in the effluent stream from the column and, if no proper action is taken, would be direct to waste or would be collected together with other non-binding components.

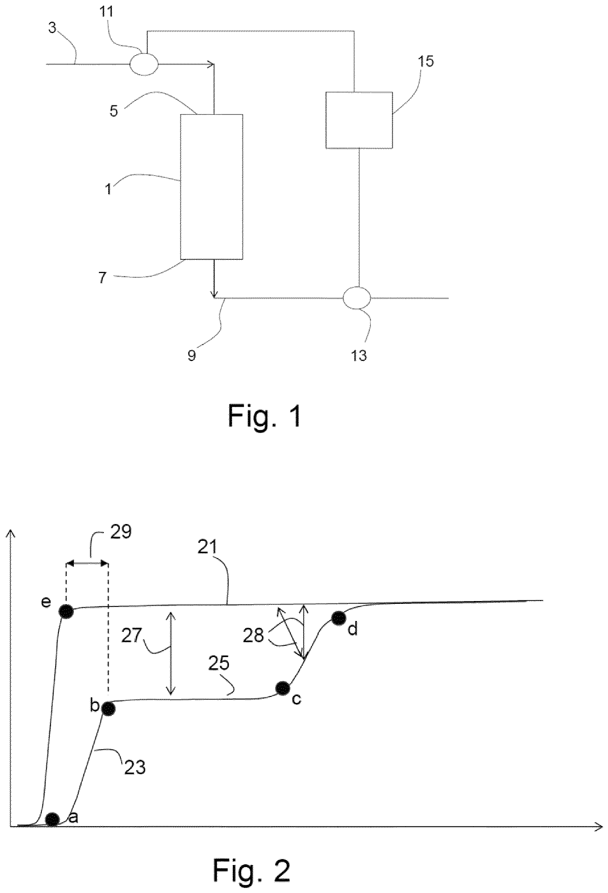

Fig. 1 shows schematically a part of a simple chromatography system according to the invention. This chromatography system comprises one chromatography column **1**. It further comprises a feed line **3** connected to an inlet end **5** of the chromatography column 1. The sample to be passed through the column **1** can be added through the feed line 3. The system further comprises an effluent line **9** connected to the opposite end, i.e. the outlet end **7** of the chromatography column **1**. The sample having passed the chromatography column **1** can pass through the effluent line **9**. The chromatography system comprises further according to the invention a first detector **11** positioned somewhere along the feed line **3**. The first detector **11** is adapted to detect a feed signal being representative of the composition of the feed material (sample) passing in the feed line. Furthermore, the chromatography system comprises a second detector **13** positioned somewhere along the effluent line **9** and adapted to detect an effluent signal being representative of the composition of the sample flowing out from the column **1** through the effluent line **9**. The first and the second detectors **11, 13** are suitably the same type of detectors and in one embodiment this is a UV detector, i.e. measuring the UV absorbance of the sample. Other possible types of detectors are measuring pH, conductivity, light scattering, fluorescence, IR or visible light. If the different detectors in the system not are the same type of detectors the detected signals need to be correlated when used for the further calculations according to the invention.

Furthermore, according to the invention, the first and second detectors **11, 13** are both connected to a determining unit **15**. Said unit analyzes the signals detected in the first and second detectors **11, 13** in order to determine the operational status of the chromatography column. Possible signals from the first and second detectors **11, 13** are shown in Figure 2 in a diagram showing signal strength over time. The feed signal is denoted **21** and is the signal from the first detector **11**. It is essentially a straight line since the feed sample is in this case and during this time window constant in composition. However, the feed signal may vary due to variations in concentration of target product and/or background.

The effluent signal is denoted **23** and is the signal from the second detector **13**. The effluent signal **23** will start rise from zero at point a, as soon as some of the sample has passed the column **1** and entered the passage of the effluent line **9** where the second detector **13** is positioned. The signal will then rise until point **b**, where it levels out into a plateau **25**. This plateau **25** arises when all the non-binding components in the feed have passed the column. A breakthrough point **c** is further defined after the plateau **25** when the signal **23** starts to rise again. This is due to the fact that the chromatography media in the column **1** starts to get saturated and some of the parts of the sample that should have been bound in the column start to break through the column. A breakthrough point d is further defined as the signal **23** approaches the signal **21**. This point is defined as a saturation point and represents the moment when chromatography media is almost fully saturated with the binding components of the sample.

According to the invention at any given point in time a Delta signal is calculated which is defined to be the feed signal **21** chosen from signals measured between the given time reduced by a specified time delay owing to the specific hardware configuration and the given time minus the effluent signal **23** measured at the given time. The feed signal **21** measures the feature (in one embodiment UV absorbance) for both non-binding and binding components of the feed. The time delay is defined as a time for a non-binding compound in the sample to travel from the feed detector **11** (Figure 1) to the effluent detector **13** (Figure1). The time delay can be measured applying the residence time distribution theory, or it can be measured by subtracting the time when signal **21** first reaches the highest plateau e from the time when signal **23** reaches the plateau **25**. This is illustrated as arrow **29** in Figure 2. The delta signal at one specific point in time is illustrated as arrow **28** in Figure 2. As indicated this arrow **28** can be inclined if the time delay is compensated for.

According to the invention a Deltasignalmax **27** is calculated which is defined to be the feed signal **21** minus the signal level for the effluent signal **23** when it is in the plateau **25**. This Deltasignalmax **27** can then be used for defining suitable levels for the breakthrough point and the saturation point for example. The breakthrough point c can suitably be defined to be a certain predefined percentage of the Deltasignalmax, for example somewhere in the span of 1-10 % or more suitably in the span of 1-3 % and the Saturation point **d** can suitably be defined to be a certain predefined percentage of the Deltasignalmax, for example somewhere in the span of 60-90 % or more suitable in the span of 70-80%.

One advantage with this approach of determining the breakthrough point and the saturation point is that this could be done automatically in real time and it is independent of the feed concentration and/or composition since the feed signal is automatically compensated for.

In a further aspect of the invention these determinations of operational status, e.g. binding capacities, such as breakthrough and saturation points, are used for automatically controlling the start and stop of the different chromatography process steps, i.e. when a certain breakthrough or saturation point level has been reached a control system can control the chromatography system to proceed to the next process step such as redirecting column effluent to a different collection point, or to stop loading step and initiate column wash step.

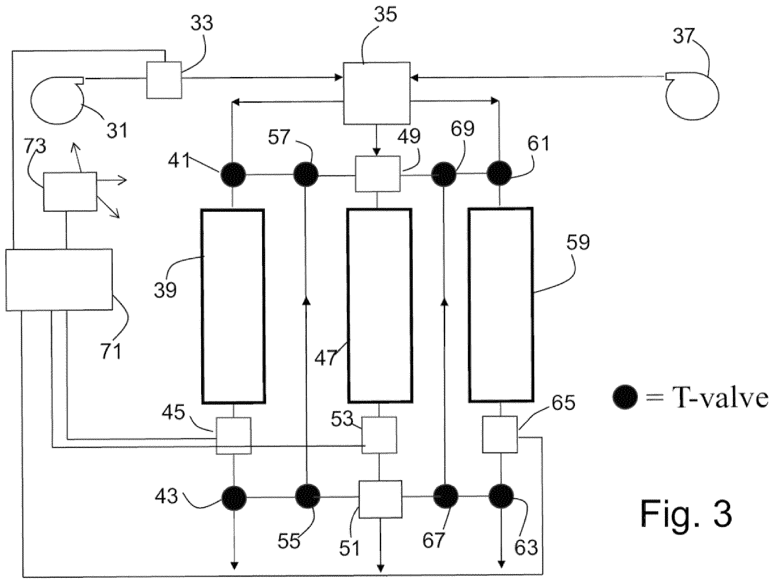

In another aspect of the invention the chromatography system comprises more than one chromatography columns, in a so called periodic counter current (PCC) system. In the periodic counter current system, most of the time the feed is passed through at least two columns connected in series. The series is often called a loading zone and addition and removal of columns in/from the loading zone is based on predetermined breakthrough and saturation points for the last and the first column in series, respectively. In Figure 3 such a system according to the invention comprising three columns is shown schematically. The benefits of the invention are even more explicit in this example, because one common problem in PCC systems is that in order to be able to get an efficient system operation the columns used in the system need to be as identical as possible and the feed composition and flow rates should be constant, or at least their changes with process time should be known a priori. With the invention, any differences in columns operational status and/or flow rates can be compensated for by adjusting for how long, and in which position, the different columns should be in the loading zone according to the determined breakthrough and saturation points.

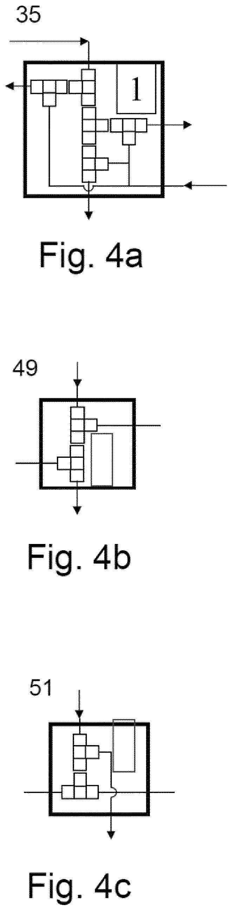

In Figure 3 a feed pump **31** is shown connected via a first detector **33** to a first valve block **35.** A buffer pump **37** is also connected to this first valve block **35.** The first valve block **35** is further connected to the inlet of a first column **39** via a first T-valve **41.** An outlet end of the first column **39** is connected to a second T-valve **43** through a second detector **45.** The first valve block **35** is further connected to the inlet of a second column **47** via a second valve block **49.** An outlet end of the second column **47** is connected to a third valve block **51** via a third detector **53.** Furthermore, a third T-valve **55** is connected between the second T-valve **43** and the third valve block **51.** The third T-valve **55** is also connected to a fourth T-valve **57** which is also connected to the first T-valve **41** and the second valve block **49.** Hereby the effluent from the first column **39** can be directed to the inlet of the second column **47** through T-valves 43, 55, **57** and block valve **49.** Furthermore, the first valve block **35** is connected to the inlet of a third column **59** via a fifth T-valve **61.** An outlet end of the third column **59** is connected to a sixth T-valve **63** via a fourth detector **65.** Furthermore, a seventh T-valve **67** is connected between the third valve block **51** and the sixth T-valve **63.** The seventh T-valve **67** is also connected to an eighth T-valve **69** which is also connected to the second valve block **49** and the fifth T-valve **61.** Hereby the effluent from the second column **47** can be directed to the inlet of the third column **59.** The effluent from the third column **59** can be directed to the inlet of the first column **39** through valves 63, 67, **51 55, 57** and **41.** The construction of the first valve block **35** is schematically shown in Figure 4a, the construction of the second valve block **49** is schematically shown in Figure 4b and the constructions of the third valve block **51** is schematically shown in Figure 4c. In these Figures each group of four boxes represents a T-valve (3 way valve). Furthermore, according to the invention the first, second, third and fourth detectors 33, 45, 53, 65 are all connected to a determining unit **71.** The determining unit is adapted to use the detected signals from the detectors to determine breakthrough and saturation points for the three different columns. The determining unit **71** and all the valve blocks and T-valves and pumps are further connected to a control unit **73** (all the connections are not shown in the Figure) which is adapted to control the chromatography system in terms of when to remove or add columns from/into the loading zone, change flow rates, start new wash steps, etc. The detectors **33, 45, 53, 65** are in one embodiment UV detectors. Other examples of detectors that can be used for this invention have previously been discussed.

In one embodiment of the invention the chromatography process carried out in the system of Figure 3 comprises:

(a) Continuously monitoring a signal in the feed line with the first detector **33** and in effluent from each of the columns **39, 47, 59** (with the second, third and fourth detectors **45, 53, 65**) and calculating a difference between the feed signal shifted back in time by a predefined delay time and the effluent signals in the outflow line from each column **39, 47, 59;**

(b) passing feed comprising at least one target compound across a 1st adsorbent (chromatography media in the first column **39),** and directing the outflow from the 1st adsorbent to a 2nd adsorbent (chromatography media in the second column **47)** when the Deltasignal (definition as described above in relation to Figure 2) measured between the feed line and the effluent from the 1st adsorbent reaches a predetermined value x1;

(c) redirecting the feed to the 2nd adsorbent, and passing wash liquid across the 1st adsorbent to which target compound has bound when the Deltasignal measured between the feed line and the effluent from a 1st adsorbent reaches a predetermined value x2;

(d) directing the wash liquid outflow to the 3rd adsorbent (chromatography media in the third column **59)** and subsequently directing the outflow from the 2nd adsorbent to the 3rd adsorbent when the Deltasignal measured between the feed line and the effluent from a 2nd adsorbent reaches a predetermined value x1;

(e) regenerating the 1st adsorbent;

(f) redirecting the feed to said 3rd adsorbent, and passing wash liquid across the 2nd adsorbent to which target compound has bound when the Deltasignal measured between the feed line and the effluent from a 2nd adsorbent reaches a predetermined value x2;

(g) directing the wash liquid outflow to the 1st adsorbent, and subsequently directing the outflow from the 3rd adsorbent to the 1st adsorbent when the Deltasignal measured between the feed line and the effluent from a 3rd adsorbent reaches a predetermined value x1;

(h) regenerating the 2nd adsorbent;

(i) redirecting the feed to said 1st adsorbent, and passing wash liquid across the 3rd adsorbent to which target compound has bound when the Deltasignal measured between the feed line and the effluent from a 3rd adsorbent reaches a predetermined value x2;

(j) directing the wash liquid outflow to the 2nd adsorbent, and subsequently directing the outflow from the 1st adsorbent to the 2nd adsorbent when the Deltasignal measured between the feed line and the effluent from a 1st adsorbent reaches a predetermined value x1;

(k) regenerating the 3rd adsorbent;

(l) repeating steps (b)-(k);

wherein at least one target compound is collected in step (d), (g) and/or (j).

Predetermined values of x1 and x2 represent breakthrough and saturation points, respectively.

The current invention enable use of not identical columns when operating a counter current system because any differences in the columns properties can be compensated for by automatically adjusting breakthrough and saturation switching points based on the Deltasignal and Deltsignalmax measured for each of the columns. It also enables operating a counter current system when unexpected changes in feed concentration occur as any change in the feed concentration, and thus a change in the mass loaded into each column can be compensated for by automatically adjusting the breakthrough and saturation switching points based on Deltasignal and Deltasignalmax that automatically compensates for variation in feed concentration.

In another embodiment of the invention, the chromatography system comprising of more than 2 chromatography columns can be used for direct capture of a product from a feed stream originated from a perfusion cell culture. For a person skilled in art, it is well known that concentrations of components in such stream will vary with time, and without an automated control algorithm operation of the chromatography system would be impossible without a risk of significant losses of product due to wrongly a priori assigned redirection points.

EXAMPLE

The present example is provided for illustrative purposes only, and should not be construed as limiting the present invention as defined by the appended claims.

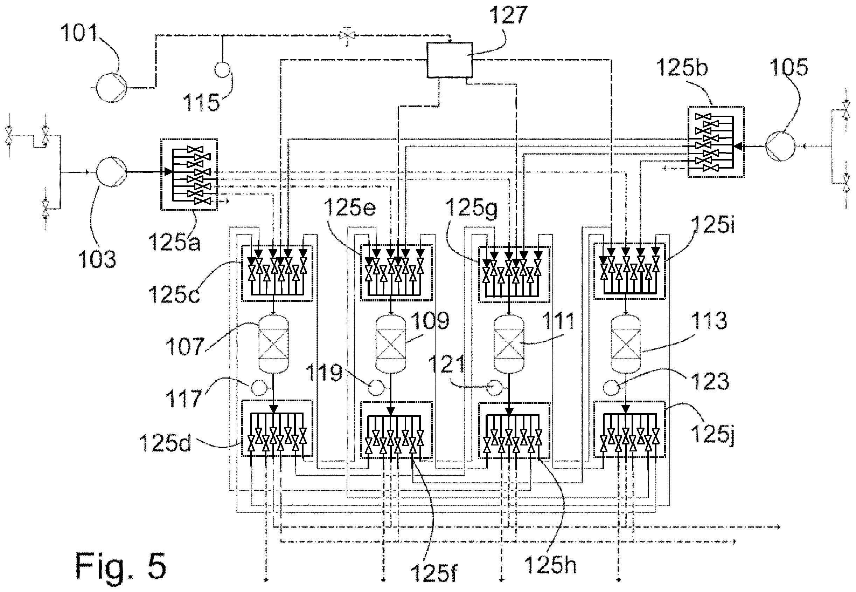

This example illustrates a continuous primary capture step for purification of a monoclonal antibody (MAb) from a mixture containing MAb and bovine serum albumin, BSA, on protein A chromatography resin using a four column periodic counter current (4C-PCC) system with deltaUVmax control according to the invention (i.e. in this example the detectors are UV detectors and the Deltasignalmax is called deltaUVmax). More specifically, four similar columns were packed with the Protein A chromatography resin MabSelect™ (GE Healthcare Bio-Sciences, Uppsala, Sweden). The columns were connected to a custom modified ÄKTAexplorer™ (GE Healthcare Bio-Sciences, Uppsala, Sweden) chromatography system (Fig. 5) that was configured into a four column periodic counter current system, 4C-PCC with an automated control function based on continuous comparison of UV signals measured before and after each of the four columns. The system comprises three independent pumps, a feed pump **101,** a first buffer pump **153** and a second buffer pump **155.** The system further comprises a first column **107,** a second column **109,** a third column **111** and a fourth column **113.** The system further comprises 5 UV detectors, a first UV detector **115** positioned on the feed line, a second UV detector **117** positioned after the first column **107,** a third UV detector **119** positioned after the second column **109,** a fourth UV detector **121** positioned after the third column **111** and a fifth UV detector **123** positioned after the fourth column **113.** The system further comprises several rotary valves **125a-j** and a flow splitter **127.** The UV detectors **115, 117, 119, 121, 123** are positioned such that a feed stream and an effluent from each of the columns was passed through a UV detector (Figure 5). Absorbance from each UV detector were recorded using UNICORN™ software (GE Healthcare Bio-Sciences, Uppsala, Sweden). UNICORN™ is also used for control of all pumps and valves. MAb eluted from the protein A columns was collected in a single pool.

The following single column chromatography cycle was used as a base for operating the 4C-PCC system in a continuous manner: 1) column equilibration with 3 column volume (CV) of buffer A; 2) column loading with feed; 3) column wash with 4CV of buffer A; 4) column elution with 4CV of buffer B; 5) column CIP with 4CV of buffer C; and, 6) column regeneration with 3 CV of buffer A. All steps were performed at 0.4 mL/min flow rate.

Composition of solutions used is given below:

Buffer A: PBS, pH 7
Buffer B: 0.1 M Sodium citrate, pH = 3.5
Buffer C: 50 mM NaOH
Feed: ~2.00g/L MAb and 3 mg/ml BSA (Sigma) dissolved in buffer A

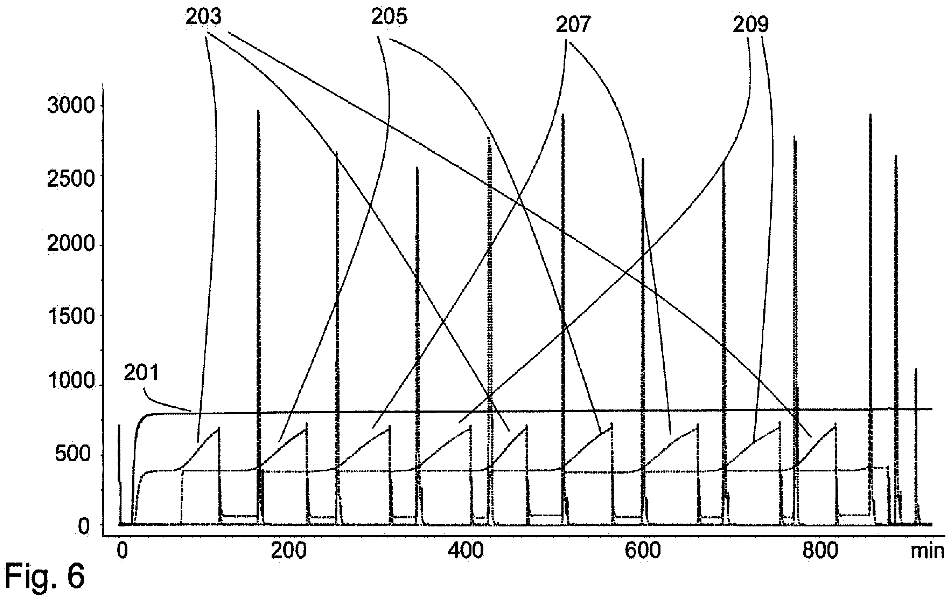

A few hundred milliliters of solution containing the feed were continuously fed into the experimental 4C-PCC setup described above. The absorbance of the feed solution was measured continuously by the first UV detector **115** positioned on the feed line (Figure 5). The purified MAb was eluted from the system in a discreet manner by applying the buffer B into the saturated column. The saturated column was washed prior to the elution step. In Figure 6, signals recorded in the feed line and in the effluent from each of the chromatography columns **107, 109, 111, 113** during the first, a start-up, 4C-PCC cycle consisting of: 1) loading of the feed into the first column **107;** 2) connecting the 1st and the 2nd columns **107, 109** in series; 3) directing the feed into the 2nd column **109** while washing the 1st column **107** into the 3rd column **111;** 4) connecting the 2nd and the 3rd columns **109, 111** in series while applying the feed; 5) eluting the 1st column **107;** 6) regenerating the 1st column **107;** 7) directing the feed to the 3rd column **111** while washing the 2nd column **109** in to the 4th column **113;** 8) connecting the 3rd and the 4th columns **111, 113** in series while continuously applying the feed; 9) eluting the 2nd column **109;** 10) regenerating the 2nd column **109;** 11) directing the feed to the 4th column **113** while washing the 3rd column **111** in to the 1st column **107;** 12) connecting the 4th and the 1st columns **113, 107** in series while continuously applying the feed; 13) eluting the 3rd column **111;** 14) regenerating the 3rd column **111;** 15) directing the feed to the 1st column **107** while washing the 4th column **113** in to the 2nd column **109;** 16) connecting the 1st and the 2nd columns **107, 109** in series while continuously applying the feed; 17) eluting the 4th column **113;** 18) regenerating the 4th column **113;** 19) repeating steps 3-18 one more time.

The repetitive UV pattern shown in Figure 2 is depicted in Figure 6, where UV signals recorded during 9 single column loadings, representing two 4C-PCC cycles, are presented. The curve denoted **201** is the signal originating from the first UV detector **115** in Figure 5, i.e. this is the feed signal. The curve denoted **203** is the signal originating from the second UV detector **117** in Figure 5, i.e. this is the effluent signal from the first column **107.** The curve denoted **205** is the signal originating from the third UV detector **119** in Figure 5, i.e. this is the effluent signal from the second column **109.** The curve denoted **207** is the signal originating from the fourth UV detector **121** in Figure 5, i.e. this is the effluent signal from the third column **111.** The curve denoted **209** is the signal originating from the fifth UV detector **123** in Figure 5, i.e. this is the effluent signal from the fourth column **113.** The chromatograms representing concentration of MAb in effluents from each of the column as shown in Figure 6 were obtained by setting deltaUV (i.e. in this example the detectors are UV detectors and the Deltasignal is called deltaUV) criteria for detection of the breakthrough and the saturation points at x = 3% and x = 70% of deltaUVmax signal, respectively, according to the principle described in this invention, where x is given by:

$$x = \frac{\text{deltaUVmax - deltaUV}}{\text{deltaUVmax}} 100\%$$

Summary of results obtained in this experiment is shown in Tables 1 and 2 below. Where the volume of feed solution and the calculated amount loaded onto each column is shown. These data are compared to the calculated results that would be obtained if no automated controlled according to this invention was used, i.e., using predesignated switching times of flow redirection as described in point 1-18 were used if the reference run were performed on the same system with the same columns. As shown in Figure 6, the UV signal measured in the effluent from the first column **107** shows that a load onto the first column was shorter before the breakthrough of MAb was observed as compared to the loads applied to the other three column until the same levels of breakthrough. This premature breakthrough indicates that either the amount of the resin in this column or the resin itself was different than in the other two columns. In fact, prior to 4C-PCC experiment the first column **107** was treated to reduce its binding capacity and by doing so making it different from the other three columns.

Because the first column **107** was different than the second column **109** a premature breakthrough for this column would have been observed in an experiment when the 4C-PCC system was operated based on predefined column switching times. Under such conditions, a loss in MAb during step 1 of the cycle would have been observed. In addition, the amount of MAb loaded onto the second column **109** in step 2 would be larger than calculated and subsequently some of the MAb would be lost in the effluent from the second column **109** before this column would be connected to the third column **111** in step 4. This mismatch in amounts loaded onto the columns would then propagate through steps 5-18, and the amount of MAb not capture by the columns would steadily increase with each column switch. This loss in unbound MAb was avoided by implementing the control algorithm according to the current invention. In Table 1, estimated masses of MAb loaded onto each of the columns during the experiment are shown. The masses loaded were estimated based on the areas above respective UV curves measured in the effluent lines after each column. The mass washed out from one column and loaded on the second to the next column in series was neglected. As shown in Table 1 significantly different mass of MAb was loaded on to the first column **107** as compared to the mass loaded on the other three columns. The mass loaded on the first column **107** was between 20 - 30% smaller than the mass loaded on each of the other columns during two different cycles that was almost the same with no more than 5% difference between columns and cycles. If on the other hand, the 4C-PCC system was operated without the automated control with a switch time set to enable load of 77 mg per milliliter resin, the amount of MAb lost per cycle and column would be significant (Table 2) and would account for around 10% of mass loaded on to the system during the whole experiment.

Table 1

| Summary of 4C-PCC run. Mass of MAb in milligrams loaded on each columns during the two loading cycles.* | | | |
|---|---|---|---|
| | Load 1 | Load 2 | Load 3 |
| First column | 54.3 | 56.6 | 55.8 |
| Second column | 77.1 | 74.2 | - |
| Third column | 74.4 | 76.5 | - |
| Fourth column | 73.8 | 74.9 | - |
| * The first column was loaded three times | | | |

Table 2. Estimated mass that would be lost (found in the waste stream) per cycle and column if the 4C-PCC was operated at the constant switching time equivalent to the load of 77 g per liter resin.

|  | Load 1 | Load 2 | Load 3 |
|---|---|---|---|
| First column | 22.7 | 20.4 | 21.2 |
| Second column | -0.1 | 2.8 |  |
| Third column | 2.6 | 0.5 |  |
| Fourth column | 3.2 | 2.1 |  |

A standard ultraviolet (UV) detector for liquid chromatography measures the absorbance of monochromatic light of fixed wavelength in the UV or visible wavelength range (typically between 190 nm and 400 nm) against a reference beam and relates the magnitude of the absorbance to the concentration of a compound in the sample passing through a flow cell contained within the detector.

Compounds suitable for UV detection typically contain unsaturated bonds, aromatic groups, or functional groups containing heteroatoms, which contain $\pi^*$ and $\sigma^*$ nonbonding orbitals into which electrons are promoted to absorb the incident energy. These nonbonding orbitals contain a wide distribution of vibrational and rotational energy levels that lead to a distribution of absorbance energies and therefore spectra with broad, rather than sharp features. Compound concentration can be determined from the Beer-Lambert law as described in more detail below.

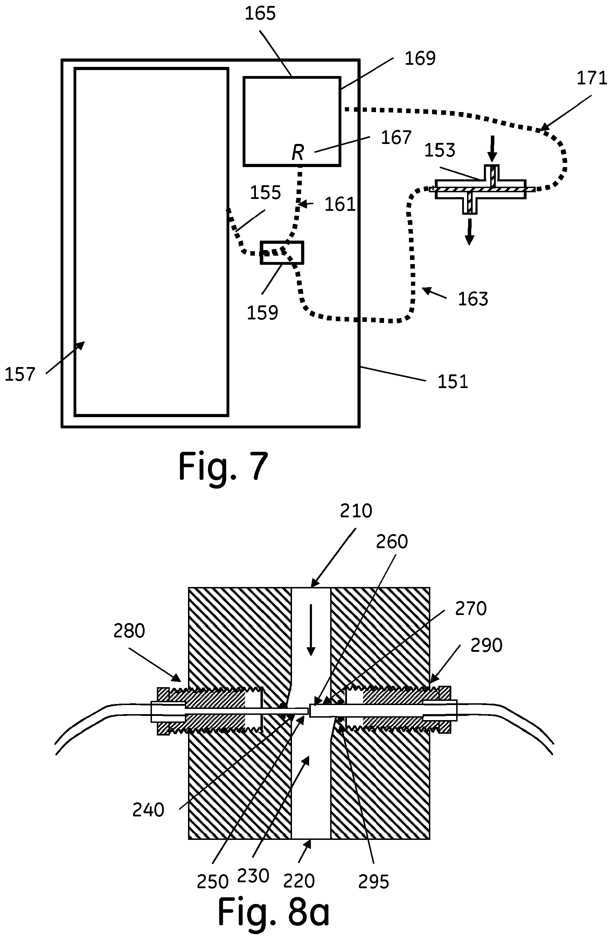

FIG. 7 illustrates a one embodiment of a flow cell absorption monitor system, such as a multi-wavelength Ultra-violet (UV)-Visible monitor, as disclosed in US 8,649,005 assigned to GE HEALTHCARE BIO SCIENCES AB.

This monitor 151 includes an interchangeable flow cell 153 and optical fibers 155 connecting the flow cell to the monitor unit 151. Monitor 151 may e.g. be a Monitor UV-900 manufactured by GE Healthcare, Life Sciences located in Uppsala, Sweden. This monitor utilizes advanced fiber optic technology to monitor light with high sensitivity at up to three wavelengths simultaneously in a range of 190-700 nm. The use of fiber optics together with the unique flow cell design ensures a signal-to-noise ratio with minimal drift and refractive index sensitivity. Typically, the monitor 151 includes a monochromator 157 with a light source (not shown), such as a xenon flash lamp (not shown) that provides a high intensity, continuous spectrum of light, and a tunable monochromator arrangement (not shown) for selecting the wavelength of light output to the fiber 155. The lamp is activated only during the chromatographic run, ensuring that its long lifetime of approximately 4000 hours of effective operation is used efficiently. However, the tuneable monochromator, may be any suitable type, capable of providing the desired wavelength range, or even two or more units with complementary wavelength ranges. In one embodiment, the tuneable monochromator is a tuneable laser unit capable of providing monochromatic light over a specified range. In some embodiments, when tuneability is of less importance, the tuneable monochromator may be substituted by one or more discrete monochromatic light sources. In another embodiment, the monochromator is replaced by a broad band light source, and a light detection unit 165 is e.g. a spectrometer capable of spectroscopically dividing the received spectra. In the disclosed monitor 151, the optical fiber 155 optics leads the light from the monochromator 157 to an optical splitter unit 159 splitting the light to a reference fiber 161 and a flow cell fiber 163 leading directly to the flow cell 153 and focus its full intensity on the liquid flow path, thus maximizing the sensitivity of the monitoring. Flow cell 153 may have any path length, such as a path length of 2mm and cell volume of 2 μl or path length of 10mm and a cell volume of 8 μl. The transmitted light through the flow cell 153 is guided to a light detection unit 165 via an optical fiber 171. The light detection unit 165 has a flow cell input 169 connected to fiber 171 and a reference input 167 connected to the reference fiber 161. The detection unit 165 further may comprise suitable processing means for comparing the flow cell input with the reference to detect changes in light absorption in the flow cell.

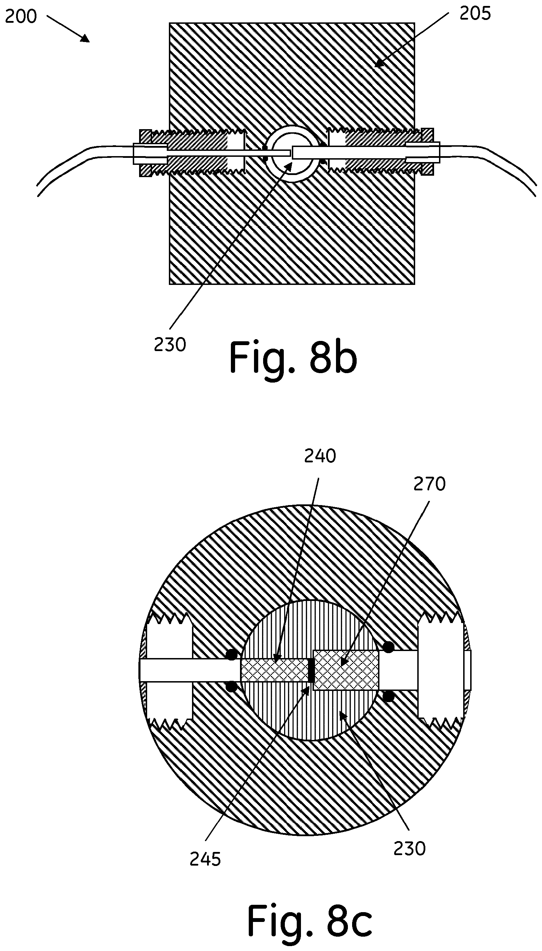

FIGS. 8A and 8B show a schematic optical flow cell detector 200 in accordance with an embodiment of the invention. The optical flow cell detector 200 comprises a detector body 205 with a sample inlet 210 and outlet 220 in fluidic communication through a flow cell channel 230 of cross sectional area A. The flow cell channel 230 may essentially be of any cross sectional area A as required for the specific application where it is to be used, depending on factors as flow rate, viscosity etc. In the disclosed embodiment, the cross sectional shape is circular which is beneficial from a manufacturing point of view as well as a fluid flow point of view, but it may actually be of essentially any suitable shape as long as the fluid flow characteristics are preserved within the desired range.

The detection optics is comprised of an input light guide 240 with a light exit surface 250 arranged adjacent and in optical alignment with a light entrance 260 surface of an output light guide 270. As is disclosed, the input light guide 240 and the output light guide 270 protrudes into the flow cell channel 230 so that the optical gap between the light exit surface 250 and the light entrance surface 260 is situated centrally in the flow cell channel 230. The optical gap may e.g. be situated at any position in the flow cell channel 230, as long as it is not located in a stagnant zone, e.g. adjacent a wall of the channel in order to ensure that the fluid in the gap is representative of the fluid flow. In the disclosed embodiment, the input light guide 240 and the output light guide 270 protrudes essentially transverse into the flow cell channel **230,** whereby exchange of fluid in the optical gap is promoted. However, the angular relationship between the fluid flow channel and the light guides **240** and **270** need not be transverse, but can be any suitable angle and configuration provided that desired renewal of the fluid in the optical gap is achieved. In alternative embodiments, the fluid flow channel **230** need not be straight, but it may be curved or the like.

It has surprisingly been found that the present approach with relatively small sized light guides **240** and **270** protruding into the fluid flow channel **230** provides excellent capability of measuring high concentrations of e.g. proteins in the flow when arranged at a short distance from each other such as 0.5 mm or less. As will be discussed in more detail below, a high degree of linearity for high concentrations has been disclosed for embodiments with an optical gap of 0.12 and 0.07 mm. The optical gap is selected depending on the concentration range to be measured, and it may e.g. be any value from 0.02 to 2 mm such as 0.7, 0.5, 0.4, 0.3, 0.2, 0.1, 0.07, 0.05 mm or there between. It has been found that the fluid in the optical gap is readily exchanged even at the smallest gaps and at moderate flow rates, which is believed to be a result of the small optical cross sectional area and the "protruded" position of the gap essentially at the center of the fluid flow channel.

In figs. 8A and 8B, the optical gap is adjustable in that the light guides **240** and **270** are arranged in threaded ferrules **280** and **290** respectively, and the optical gap may be adjusted by turning the ferrules **280** and **290.** In figs. 8A and 8B, the light guides **240** and **270** are sealed from the fluid flow channel by seals **295** e.g. in the form of O-rings or the like, to avoid fluid form exiting there through.

As is mentioned, it is believed to be essential to avoid disruption of the fluid flow, whereby a small cross sectional area of the protruding portions of the light guides and the output light guide in the flow direction is essential, and in the disclosed embodiments the total cross sectional area of the protruding portions of the input and output light guides **240, 270** is less than about A/2, or even A/4 in the flow direction. Fig. 8C shows an enlargement of the flow cell channel **230** region of the flow cell detector **200** of fig 8A and 8B, wherein the area A of the flow cell channel **230** is filled by a broad diagonal pattern and the protruding portions of the input and output light guides **240, 270** are filled by a rombohedric pattern. From fig 8C it is clear that the uninterrupted area of the flow cell channel **230** is essentially larger than the total area of the protruding light guides **240** and **270,** whereby the fluid flow in the flow cell channel will be relatively uninterrupted. As mentioned the dimensions of the protruding sections of the input and output light guides **240, 270** may be designed in any suitable way to achieve a small cross sectional area compared to the area A of the flow channel **230,** whilst providing rigid positioning of the optical end-surfaces of the input and output light guides **240, 270** respectively, in order to achieve desired optical transmittance there between. In fig. 8C the optical cross sectional area or the "detection area" between the input and output light guides **240, 270** is indicated by dashed region **245**. As is concluded elsewhere, the optical cross sectional area **245** in the present flow cell detector **200** is very small compared to the total area A of the flow channel **230,** e.g. less than A/50, A/100, A/200, A/1000 or less. However, the present light guide type design ensures a high light flux in the small optical cross sectional area **245** (volume).

The light guides may be comprised of any suitable optical material capable of transmitting light, such as glass, quarts, light transmitting polymers etc. One or both may be provided with an optically blocking sleeve or coating (not shown) at the peripheral surface to avoid leakage of light. The light guides **240** and **270** may be cylindrical with a diameter of 5 mm or less, e.g. 3 mm, or 1 mm or the like. The diameter of the light guides **240** and **270** obviously depends on the total area A of the flow channel **230**. The cross sectional flow area of the fluid flow channel, schematically disclosed in figs. 8A and 8B, may be essentially uniform in the flow direction throughout the flow cell. The reduced cross sectional flow area due to the protruding light guides **240** and **270** is compensated by a local widening of the fluid flow channel. This is particularly useful for flow cells with a relatively small fluid flow channel compared to the protruding light guides, but in other embodiments no compensation may be needed.

As can be seen in figs. 8A and 8B, the light guides **240** and **270** may be asymmetric in core size, with the input light guide being thinner than the output wave guide, in order to capture essentially the whole cone of light (its numerical aperture) emerging from the light exit surface at the light entrance surface **260**.

In Figs 8A and 8B, the light guides **240** and **270** are shown in optical contact with optical fibers **163** and **171** respectively, in turn connected to a monitor unit **151** (not shown). However, one or both of the light guides **240** and **270** may be directly coupled to the optical detection system of the flow cell monitor system **151.**

UV cell pathlength of the detector defines an operating window having an upper limit, and wherein the step of adapting the UV cell pathlength further comprises reducing the UV cell pathlength while maintaining the estimated maximal concentration of target compound below the upper limit.

The concept behind the invention is to provide a method for determining operational status, e.g. binding capacities, of a chromatography column, comprising:

- detecting a feed signal representative of the composition of a feed material provided to the inlet of the column;
- detecting the UV absorbance in the feed material,
- detecting an effluent signal representative of the composition of the effluent from the column;
- using the feed signal and the effluent signal to determine operational status of the column.

The feed signal is generated using a first UV detector having a first UV cell pathlength operating at a first UV wavelength and the effluent signal is generated using a second UV detector having a second UV cell pathlength operating at a second UV wavelength. The method further comprises.

- determining a first threshold value based on the detected UV absorbance in the feed, and
- selecting the first UV cell pathlength and/or first UV wavelength based on the first threshold value.

The advantage of the above mentioned features is that the possibility to measure the composition of the feed material even when the composition of feed material varies over time, or is higher than originally anticipated.

The step of determining the first threshold value may comprise:

- estimating maximum UV absorbance in feed material, and
- selecting the first threshold value to ensure that the estimated maximum UV absorbance in the feed material is detectable in the first UV detector.

The feed material comprises impurities and product, and the absorbance measured by the first UV detector is a combination of the contribution from both. The impurities results in background absorbance when measured in the first UV detector, and the product results in an absorbance level of the product in the feed material when measured in the first UV detector.

In one aspect the absorbance level of the product is at least forty percent (40%) of the background absorbance of the feed material. In another aspect, the absorbance level of the product in the feed material is higher than the background absorbance, preferably the absorbance level of the product in the feed material is 70%-90% of the absorbance measured in the first UV detector.

The step of selecting the first UV cell pathlength may comprise selecting a fixed UV cell pathlength from a plurality of pre-selected UV cell pathlengths, or adjusting the UV cell pathlength. However, the step of selecting the first UV wavelength may also comprise adjusting the first UV wavelength.

According to one aspect, the step of selecting the first UV cell pathlength and/or first UV wavelength is performed until a suitable operating window of the first UV detector is achieved. This is achieved when the first UV detector is within a linear range of detection.

In a first embodiment, the UV absorbance of the feed material is calculated, and the step of estimating maximum UV absorbance is based on the calculated UV absorbance. The calculation may be based on the Beer-Lambert Law (see above).

In a second embodiment the UV absorbance of the feed material is measured, and the step of estimating maximum UV absorbance is based on the measured UV absorbance. The measurement may be performed before running a batch in the chromatography system to calibrate the UV detector. Alternatively, the UV absorbance of the feed material is continuously measured, and the first threshold value is continuously selected based on changes in UV absorbance.

The step of selecting the first threshold value may be performed when the change in UV absorbance is more than a pre-determined percentage compared to the first threshold value. As an example, the pre-determined percentage may be 1%, 2%, 5%, 10%, 15% or 20% of the threshold value used to select the UV cell pathlength. In a particular embodiment, the UV detector comprises a plurality of pre-selected UV cell pathlengths. This may be realised by a UV detector provided with several parallel light guides having different UV cell pathlengths. In another embodiment, the distance between the light guides may be adjusted to obtain different UV cell pathlengths in response to desired characteristics.

Thus, if the UV detector measure an increase in UV absorbance of a pre-determined percentage, e.g. 5%, then the UV cell length is reduced to ensure that the composition of the feed material still can be detected.

The method further comprising detecting the feed signal and the effluent signal using the same type of detector, i.e. UV detectors, to detect the UV absorbance in the effluent. The effluent signal is generated using a second UV detector having a second UV cell pathlength operating at a second wavelength, the method further comprising:

- determining a second threshold value based on the detected UV absorbance and/or the first threshold value, and

- selecting the second UV cell pathlength and/or second UV wavelength based on the second threshold value.

Preferably, the second threshold value is lower or equal to the first threshold value.

According to one embodiment, the step of determining the second threshold value comprises:

- estimating UV absorbance for a selected breakthrough point in the effluent, and

- selecting the second threshold value to ensure that the estimated UV absorbance for the selected breakthrough point is detectable in the second UV detector. The selected breakthrough point is normally defined as a certain percentage of the deltasignalmax, as previously described.

The breakthrough point is an indication that the chromatography column is full and the feed of material into the column has to stop or be shifted to another column.

The UV absorbance for the selected breakthrough point may be calculated, and the step of estimating UV absorbance for the selected breakthrough point in the effluent is based on the calculated UV absorbance for the selected breakthrough point.

In a further embodiment the method further comprises measuring the UV absorbance in the effluent, preferably continuously, and the step of estimating UV absorbance for the selected breakthrough point is based on the measured UV absorbance.

In an embodiment, the method further comprises selecting the second UV cell pathlength to be equal to the first UV cell pathlength.

Similar to the first UV detector, the step of selecting the second UV cell pathlength may comprise selecting a fixed UV cell pathlength from a plurality of pre-selected UV cell pathlengths, or adjusting the UV cell pathlength. However, the step of selecting the second UV wavelength may also comprise adjusting the second UV wavelength.

According to one aspect, the step of selecting the second UV cell pathlength and/or second UV wavelength is performed until a suitable operating window of the second UV detector is achieved. This is achieved when the second UV detector is within a linear range of detection.

The method may further comprise, when the first UV cell pathlength and/or first UV wavelength is changed, applying a time delay corresponding to a residence time in the column before the second UV cell pathlength and/or second UV wavelength is changed.

Advances in cell culture technology have enabled the production of antibody titers upwards of 30 g/L. These highly productive cell culture systems can potentially lead to productivity bottlenecks in downstream purification due to lower column loadings, especially in the primary capture chromatography step. Alternative chromatography solutions to help remedy this bottleneck include the utilization of continuous processing systems such as periodic counter current chromatography (PCC).

Recent studies have provided methods to optimize and improve the design of PCC for cell culture titers up to 5 g/L. This disclosure defines an experimental strategy for purification of a range of cell culture titers up to about 41 g/L. Initial experimentation showed an inability to determine a difference in change in UV280 nm signal (i.e. ΔUV) between cell culture feed and monoclonal antibody (mAb) concentration for column loadings up to at least 100 g/L. Further investigation revealed UV280 absorbance of the cell culture feedstock without antibody was outside of the linear range of detection. Additional experimentation showed the difference in ΔUV for various cell culture feedstocks can be either theoretically predicted by Beer's Law given a known absorbance of the media background and impurities or experimentally determined using various UV280 cell pathlengths. Based on these results, a 0.35 mm pathlength was chosen for dynamic control. Dynamic binding experiments showed a difference in experimental versus theoretical antibody breakthrough curves that was heavily dependent on the feed type and shape of breakthrough chromatograph. Based on the DBC and ΔUV data, the breakthrough challenge for column experiments was set at ΔUV of 70%. Performance metrics, yield, and purity, were acceptable between various cell culture feeds and titers up to about 31g/L, which showed ΔUV can dynamically control and define loading in a continuous chromatography process. At titers above 31 g/L, the linearity declines for a 0.35 mm UV pathlength at a UV280 nm signal but retains linearity at a UV300 nm signal using the same pathlength. Therefore, the UV300 nm signal could be a detection option for controlling loading of feeds greater than 31 g/L. The strategy and results presented in this disclosure show column loading in a continuous chromatography step can be dynamically controlled independent of the cell culture feedstock and titer and allow for enhanced process control for implementation into downstream continuous operations.

Transition from batch to continuous processing has proven very successful in many industries, yet its implementation into biologics manufacturing has currently been relatively deliberate [1-3]. This can be attributed mainly to a perception that continuous technology is still in its infancy (i.e. an emerging technology) and due to uncertainties regarding regulatory demands for moving from batch production to continuous production [4, 5]. Additional concerns for adaptation are centered around an increased complexity in operation and process control [6]. The trend towards continuous processing, as seen in many other industries, is clear from an economic perspective since it can achieve higher productivities at shorter processing times with reduced total costs, reduced equipment footprint, and consistent product quality [7]. Continuous processing has been extensively studied and established for fermentation operations using perfusion culture technology but limited examples exist for downstream continuous processing [8-10]. For example, recent literature has demonstrated a continuous process from a perfusion bioreactor through to the downstream polishing steps using periodic counter-current chromatography for the capture and polishing chromatography steps US20140255994(A1), Genzyme Corporation. Current approaches for downstream continuous chromatography include simulated moving bed chromatography (SMB), periodic counter-current chromatography (PCC), and sequential multi-column chromatography (SMCC) [11-14]. These enabling technologies refer to a chromatography capture step, utilizing multiple columns which are periodically moved against the direction of feed flow. The main difference of each technology is manifested in the design of the actual hardware between different multicolumn chromatographic systems. One main challenge is that limited information, process controls, and methodologies exist for implementation of these chromatography technologies from laboratory to manufacturing scales to enable a continuous downstream process.

One key facet for operating a continuous chromatography process involves the determination of a steady state protein loading onto each column. Steady state loading allows for minimal protein losses in the process resulting in higher productivities compared to a batch process. Previous solutions for controlling protein loading during continuous chromatography involve either a time based or dynamic control mode. Time base mode involves protein loading as a function of a set value of time, which can result in higher protein losses as a function of resin re-use assuming a decrease in dynamic binding capacity with time [15]. In addition, time base mode for protein loading also does not take into consideration any potential variability in performance amongst columns. Dynamic control of protein loading is based on the relative difference in ultraviolet (UV) signal between the column inlet and outlet UV absorbance (i.e. ΔUV) converted into a percentage. Using this relationship allows loading to a defined level of breakthrough, in order to maximize the column loading to ensure minimal product losses and consistent protein concentrations in each chromatography elution pool [16]. One challenge with dynamic control as cited in the literature is that a minimal difference in UV signal of impurities and antibody can occur for different cell culture feedstocks, which significantly decreases the robustness and utility of dynamic control. In turn, this low signal to noise ratio forces the user to operate in a time based mode for protein loading [17]. Throughout this specification, the term protein is used and should be construed in a general manner to comprise proteins in general and to include but not be limited to antibodies, monoclonal antibodies, antibody conjugates and protein conjugates.

Previous work in the literature has demonstrated the use of multivariable wavelength detectors to control and equalize the antibody breakthrough signal given a perfusion titer of about 2-3 g/L [18]. This allows the user to achieve a consistent delta UV percentage across columns to eliminate variability in column loading [18]. One drawback of this approach for dynamic control is that the root cause of the interference problem would need to be investigated and determined at various wavelengths, which can be an intractable challenge with current cell culture media designs and continuous upstream cell culture optimization. Another challenge of a multivariable wavelength detector is the protein absorbance will be significantly reduced, which may limit the range of feed titer that can be dynamically controlled by the UV detector. Our discovery builds upon the initial principle of UV control by providing a solution for overcoming the difference in UV signal between impurities and proteins, which allows the user to utilize dynamic control for protein loading in a continuous chromatography process. Furthermore, in this disclosure, the ability to dynamically control protein loading for high concentration cell culture feedstocks up to about 31g/L is also demonstrated, and additional data indicates that dynamic control is possible for titers greater than 31g/L if a UV300nm wavelength were utilized.

Investigation of impurity versus antibody absorbance levels for a variety of different UV cell pathlengths were evaluated and compared against theoretical calculations to determine the optimal pathlength for signal detection and control. Continuous chromatograph experiments were executed using different harvest impurity feedstocks and titers to determine the ability to dynamically control column loading independent of different types of harvest feedstocks. These sets of data were utilized to define a continuous chromatography process, which can dynamically control protein loading independent of the cell culture feedstock, generate consistent quality elution pools, and allow for enhanced process control for implementation into downstream continuous operations.

MATERIALS & METHODS

Harvested Cell Culture Fluid (HCCF)

The following relating to HCCF illustrates a possible application for the chromatography system according to claim 1. However it does not form part of the present invention.

The antibodies used in this study along with cell culture media are listed in Table I.

| Antibody | Isotype | Approximate Isoelectric Point (pI) | Cell Culture Media and Impurities | HCCF Concentration Range (g/L) |
|---|---|---|---|---|
| mAb 1 | IgG4 | 6.8 | Low UV absorbance feed | 1.5 to 31 |
| | | | High UV absorbance feed (ie. tricky feed) | 3 to 26 |
| mAb 2 | IgG1 | 8.2 | Medium UV absorbance feed | 3 to 41 |
| mAb 3 | IgG1 | 9.0 | High UV absorbance feed (ie. tricky feed) | 3 to 28 |

Table I: Properties of monoclonal antibodies and cell culture characteristics used for the dynamic binding experiments.

Each fermentation batch consisted of a 14-day fed-batch process with harvest viability of at least 60%. Each batch was harvested by an optional centrifugation step followed by depth filtration and sterile filtration. The material was then stored frozen and thawed prior to use. A portion of the HCCF was ultrafiltered to reach some of the desired feed concentration set points if necessary. The UV background of the impurities was not significantly affected by this concentration step.

Protein A Chromatography Process

The chromatography resin used throughout this study was MabSelect SuRe (GE Healthcare, Uppsala, Sweden), which is an agarose-based Protein A affinity matrix. The columns were equilibrated with 3 column volumes (CVs) of 10 mM sodium phosphate pH 6.5. After loading of harvested cell culture fluid, the columns were washed with equilibration buffer for 3 CVs. To remove product-related impurities, the columns were washed with 10 mM sodium phosphate, 0.5M sodium chloride pH 6.5 for 3 CVs followed by equilibration buffer for 3 CVs. The antibody was eluted using 20 mM sodium acetate pH 3.5 for about 3-4 CVs. The columns were cleaned using a combination of 100 mM acetic acid pH 2.9 and 0.1N NaOH. All columns were stored in 200 mM sodium acetate, 2 v% benzyl alcohol pH 5.5.

Reverse Phase High Performance Liquid Chromatography (RP-HPLC)

The analytical RP-HPLC method consisted of a R2/10, 2.1 mm × 30 mm column (Applied Biosystems) with column temperature maintained at 70°C. The antibody concentration was determined using the following gradient at a flowrate of 2 mL/min: Mobile Phase A: 0.2 v% trifluoroacetic acid (TFA) in water, Mobile Phase B: 0.2 v% TFA in 90% acetonitrile, Method: 68% A to 40% A in 5 minutes followed by 1 minutes at 100% B, and then 2 minutes at 68% A. UV280 nm absorbance was recorded during each injection and peaks were integrated using Chemstation software (Agilent Technologies). Antibody concentration was determined using a standard curve.

Ultra Performance Size Exclusion Chromatography (UP-SEC)

The analytical UP-SEC method consisted of a BEH 200, 4.6 × 150 mm column (Waters) with column temperature maintained at ambient temperature (15 - 30°C). Monomer, dimer, and higher order aggregate separation was obtained in 100 mM sodium phosphate, 100 mM sodium chloride pH 7.2 with a flow rate of 0.5 mL/min for 5 minutes. UV214 nm absorbance was recorded during each injection and peaks were integrated using Chemstation software (Agilent Technologies). The monomer purity percentage was determined by the monomer peak divided by the total peak area. The aggregate content percentage was determined by the sum of the peak area of each aggregate peak divided by the total peak area.

High Performance Ion Exchange Chromatography (HP-IEX)

Analytical high performance ion exchange chromatography (HP-IEX) method was performed using a 4 mm × 250 mm Dionex cation exchange ProPac WCX-10 column (Waltham, MA, USA) on an Agilent 1200 HPLC system. The main monomer peak was separated from the acidic and basic mAb variants using a linear gradient with increasing salt concentration and pH. Acidic variants are defined as species with pl less than the average pl of the monomer whereas basic variants are defined as specifies with pl higher than the average pl of the monomer. The main peak percentage was determined using UV280 nm by dividing the main monomer peak area by the total peak area. The acidic or basic variant percentages were determined by the sum of the acidic or basic variant peak areas respectively divided by the total peak area. Acidic and basic variants can be composed of monomers and aggregates.

Ultraviolet (UV) Absorbance Measurements of HCCF

UV280 nm measurements were taken of both the HCCF feeds and samples without antibody (i.e. Protein A flowthrough) ranging from 3 to 41 g/L using pathlengths from 0.05 to 2 mm using the Solo VPE (C Technologies, Bridgewater, NJ). A curve of absorbance versus pathlength was created to determine linearity of the feeds. The theoretical antibody absorbance was also measured by Beer's Law and compared to the experimental value for various cell culture feeds. Beer's Law is described by the following equation:

$$A = E^{1\%} \times b \times c$$

where A = absorbance (Au)

$E^{1\%}$ = extinction coefficient at 1% of 280nm absorbance
b = pathlength (mm)

and c = protein concentration (g/L)

Dynamic Binding Chromatography (DBC) Experiments

HCCF was loaded onto about a 4 mL column (0.5 cm $\times$ 20 cm) containing MabSelect SuRe resin up to a resin loading of about 120 grams of protein per liter of resin (g/L) to ensure full antibody breakthrough. Fractions were collected during loading and analyzed using RP-HPLC to determine percentage of antibody breakthrough. Linear velocity ranges were studied from 100 to 600 cm/hr. For the dynamic binding experiments, antibody breakthrough percentage was calculated using the following equation:

$$BT = \frac{C_{loading\ fraction}}{C_{HCCF}} \times 100$$

where BT = antibody breakthrough (%)

$C_{loading\ fraction}$ = antibody concentration by offline RP-HPLC of each fraction during column loading (g/L)

and $C_{HCCF}$ = antibody concentration by offline RP-HPLC of HCCF (g/L)

The dynamic binding capacity was predicted for various antibody feed concentrations and linear velocities with the Method Design Tool (GE Healthcare, Uppsala, Sweden) using the pore diffusion model. Theoretical DBC results were compared against experimental DBC results to determine the accuracy of the pore diffusion model as a function of antibody feed concentration.

Periodic Counter-Current Chromatography (PCC) Experiments

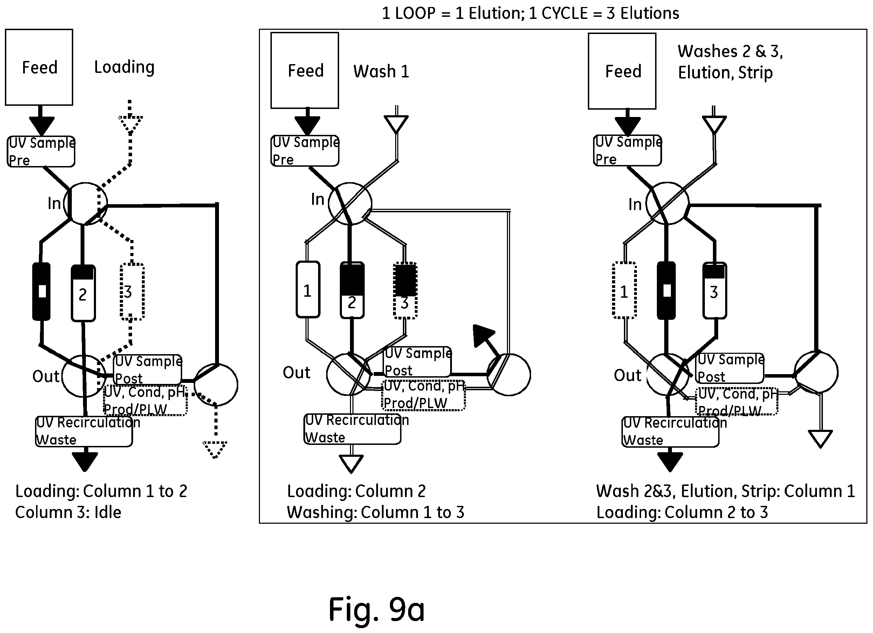

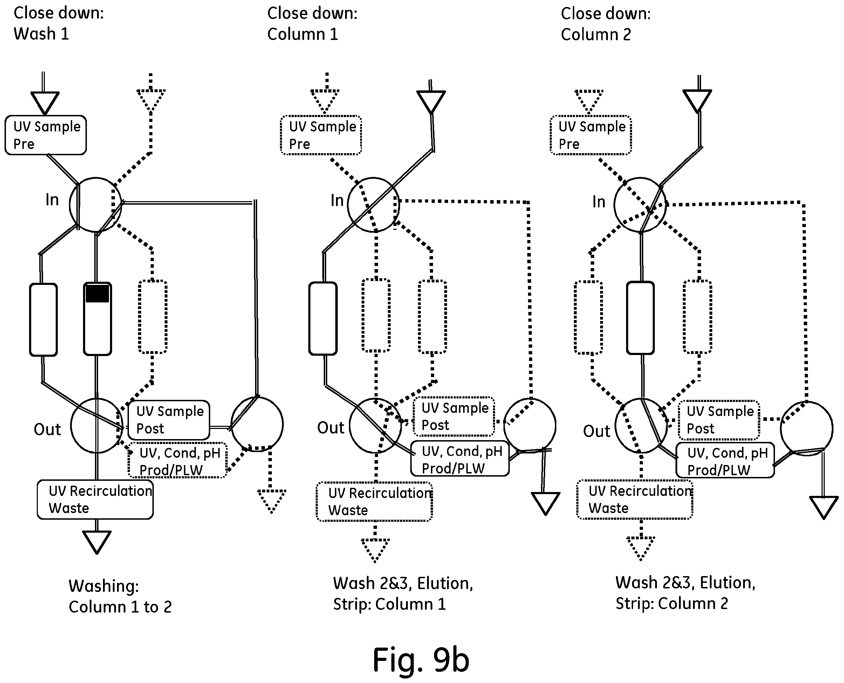

The PCC chromatography system was operated in the 3-column mode using three 4 mL columns. Figures 9A and 9B details the process description for the PCC system when operated in the 3-column mode. Figure 9A shows a schematic of the start-up phase (i.e. loading) and loop phase of the 3-column ÄKTA pcc 75 system, and Figure 9B shows a schematic of the close down phases of the 3-column ÄKTA pcc 75 system. In an effort to test the capacity of the UV cells to dynamically control the HCCF loading and maximize the resin capacity, the deltaUV was set at 70% during the HCCF loading. In the start-up phase, the HCCF was loaded onto the first column in the loading zone (Column 1) and the breakthrough (BT) was collected onto the second column in the loading zone (Column 2) until column 1 reached 70% breakthrough capacity as indicated in Figure 9A. The HCCF loading was switched to column 2 while the first wash buffer is applied to column 1 and passed to column 3 to retain any unbound protein. The second and third wash steps, elution, regeneration, and equilibration are performed on column 1 while the HCCF continues loading onto column 2 (i.e. first column in the loading zone) and the breakthrough was collected onto column 3 (i.e. the second column in the loading zone). If the deltaUV reaches 70% before the end of the loop, then the HCCF loading onto the column will stop until a new column is available. The PCC system continued to switch and load for the other columns as described until designated in the program to stop the PCC cycle (i.e. one PCC cycle is equivalent to three column elutions while one PCC loop is equivalent to one column elution). In the first phase of the closedown block, the first wash is applied onto column 1 and passed to column 2 to retain any unbound protein.

In the second phases of the closedown block, columns 1 and 2 are washed, eluted, and regenerated separately, as indicated in Figure 9B. Each elution peak is collected into one main elution pool based on a UV start trigger and CV end collection criteria. Each column is stored in 200 mM sodium acetate, 2v% benzyl alcohol pH 5.5 at the end of each PCC experiment.

Dynamic Control

The dynamic control function utilizes the difference in UV absorbance between the total UV absorbance from the product and the background, and the background of the feed. This difference is defined as $\Delta$UV.

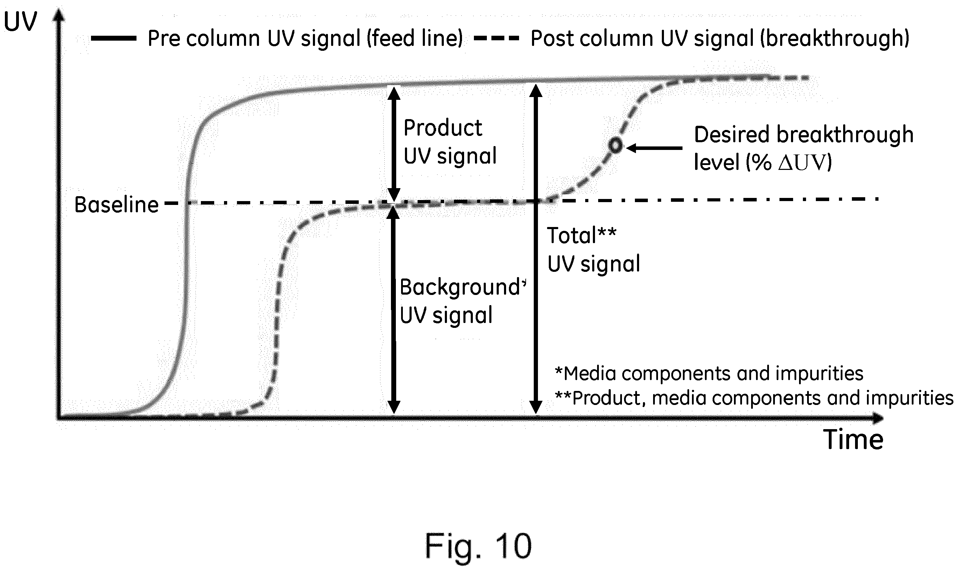

Figure 10 shows a schematic of the dynamic control function and determination of $\Delta$UV during HCCF for the ÄKTA pcc 75 system. The UV detectors responsible for the dynamic control establishes the $\Delta$UV from the difference in UV absorbance signal before the column ($UVP_{re\ column}$) and after the column ($UV_{Post\ column}$). Two additional UV detectors are also responsible for monitoring the UV after the second column in the load zone (UV Sample Recirculation) and the over the eluted column (UV Prod), not shown in the figure, respectively. For our studies, either a~2 mm or ~0.35 mm pathlength on a single UV280 nm cell was utilized to detect $\Delta$UV. Each UV cell was calibrated against the UV Prod cell prior to experimentation. The $\Delta$UV was set to 70% for the initial experiments and then ranged from about 20 - 45% for subsequent experiments.

RESULTS & DISCUSSION

Evaluation of Dynamic Binding Capacity for Various HCCF Feedstreams

Prior to testing any of the HCCF feeds with the 3-column PCC method, the relationship between antibody breakthrough as a function of column loading and $\Delta$UV was investigated. A dynamic binding capacity (DBC) experiment was conducted using the high UV absorbance HCCF to determine the $\Delta$UV difference of impurities versus antibody.

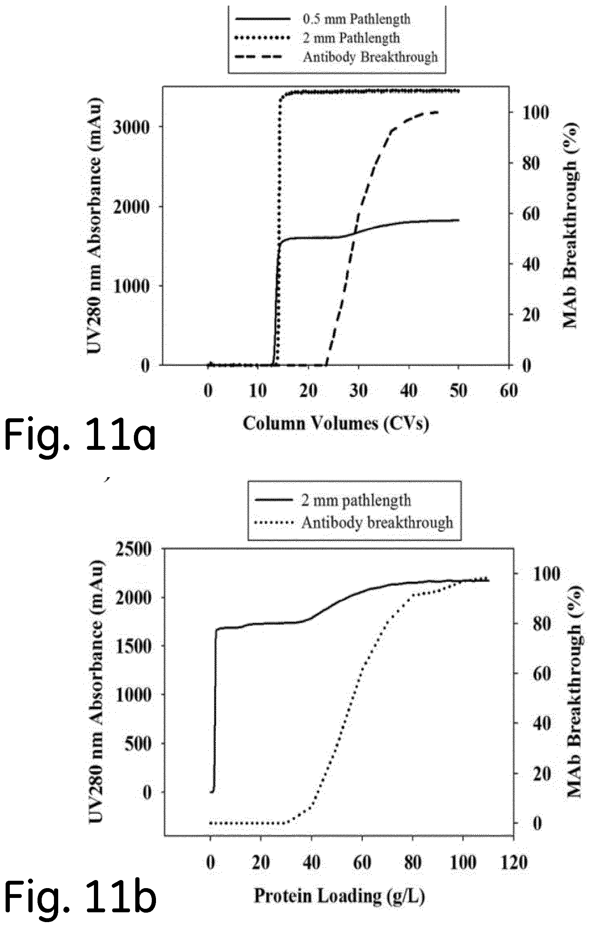

Figure 11A shows a comparison of antibody breakthrough using a 0.5 mm UV cell pathlength (solid line) versus a 2 mm UV cell pathlength (dotted line) as a function of column volumes during loading of HCCF for mAb 1 tricky feed stream. Column fractions were collected during loading and tested by RP-HPLC to determine antibody concentration (dashed line) and overlaid with the UV280 nm absorbance curve obtained from ÄKTA avant chromatographs. Figure 11B shows antibody breakthrough (dashed line) measured offline overlaid against UV280 nm absorbance at 2 mm UV cell pathlength (solid line) as a function of protein loading (grams of mAb per liter of resin) for mAb 1 low UV absorbance feed stream.

Since the media impurities had a very high background absorption outside the linear range of the UV detector, the DBC experiment did not show any difference in $\Delta$UV as indicated in Figure 11A. Therefore, it was hypothesized that a reduction in UV cell pathlength, bringing the measurement back into the linear range, result in antibody absorbance signals lower than with a 2 mm cell but still significant enough to detect breakthrough on a column.

In order to confirm this hypothesis, Beer-Lambert's Law was utilized to calculate the theoretical antibody absorbance for two different types of mAb #1 HCCF feed streams at 0.5 mm versus 2 mm UV cell pathlengths. For the high UV absorbance feed, the theoretical mAb #1 absorbance was 0.85 Au but the HCCF components plus antibody was at least 4.4 AU, which is beyond the linear range and explains why antibody breakthrough could not be detected in the DBC experiment, as indicated in Table II. However, at 280 nm and a pathlength of 0.5 mm, the theoretical antibody absorbance decreased from 0.85 AU to 0.21 AU, which should be sufficient to allow for dynamic control using $\Delta$UV. The HCCF absorbance without antibody decreased significantly within the linear range to 1.6 AU. Another DBC experiment was performed using the 0.5 mm UV280 nm pathlength and resulted in a $\Delta$UV of about 0.22 AU, which correlated exactly to the increase in antibody breakthrough detected through offline RP-HPLC analysis.

Table II: Theroetical versus experimental results for mAb #1 absorbance at 0.5 mm versus 2 mm UV cell pathlengths for two different types of HCCF.

| Type of HCCF | UV280 nm Pathlength (mm) | Cell Culture Media and Impurities Absorbance (Au) | MAb #1 Theroetical Absorbance (Au) | MAb #1 Experimental Absorbance (Au) |
|---|---|---|---|---|
| High UV absorbance feed (ie.. tricky feed) at 3 g/L titer | 2 | ≥ 3.5 (Limit of detection) | 0.85 | Beyond linear range of detector |
| | 0.5 | 1.6 | 0.21 | 0.22 |
| Low UV absorbance feed at 1.5 g/L titer | 2 | 1.3 | 0.41 | 0.42 |
| | 0.5 | 0.4 | 0.09 | 0.10 |

For the mAb #1 low UV absorbance HCCF feed stream, Beer Lambert's law indicates the theoretical antibody absorbance at 0.41 Au will be sufficient for ΔUV control at 2 mm pathlength but the theoretical antibody absorbance at 0.10 Au will be too low for accurate ΔUV control at the 0.5 mm pathlength. A DBC experiment was performed using the 2 mm pathlength cell and confirmed a ΔUV difference of 0.42 Au, which matched the calculated antibody absorbance of 0.41 Au from Beer's law, as indicated in Figure 11B. Therefore, column loading of the mAb #1 low UV absorbance feed can be dynamically controlled in a PCC mode using a 2 mm UV cell pathlength.

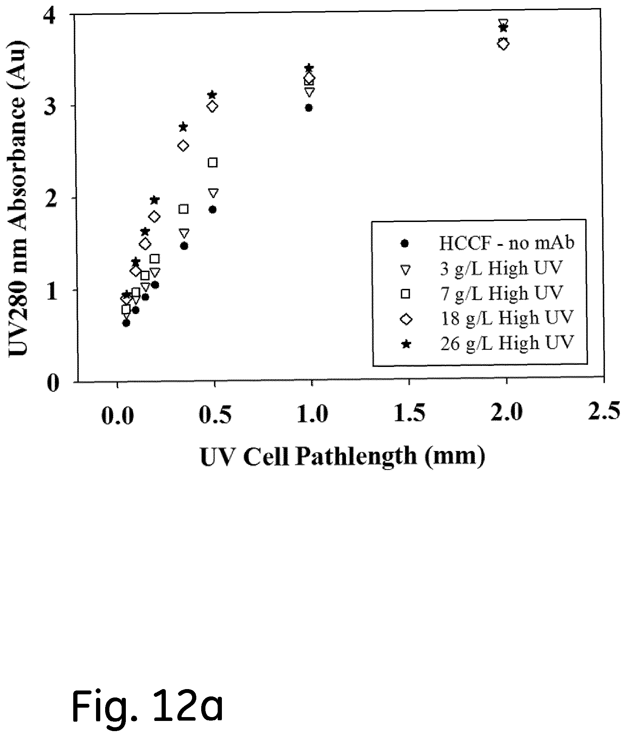

However, the column loading of the mAb #1 high absorbance feed can only be dynamically controlled using a lower UV cell pathlength, for this study a pathlength of 0.5 mm was sufficient. In order to utilize a lower UV cell pathlength in future PCC experiments, the linearity and ability to sufficiently control the ΔUV was investigated in connection with figures 12A-12D.

Determination of UV Cell Pathlength and Linearity for Dynamic Control of Various HCCF Feeds A prerequisite for reliable real time monitoring of the UV absorbance during column loading or elution is that the UV absorbance signal is within the linear range. This is not a limitation from running PCC or continuous chromatography. Rather it is an inherent characteristic of the feed used in the process, where both the antibody titer as well as the background of the impurities, media and components will influence the available range for the dynamic control. Since it was demonstrated that Beer's law can be used to predict if ΔUV control will be successful for different HCCF feed streams for a given antibody, the absorbance of different HCCF feedstocks for various antibodies were measured to determine both the ΔUV difference and linearity versus UV cell pathlength. This information will provide valuable insight to determine if the column loading of different HCCF feedstocks at various titers from 3 to 41 g/L can be dynamically controlled in a PCC mode, as indicated in Table I.

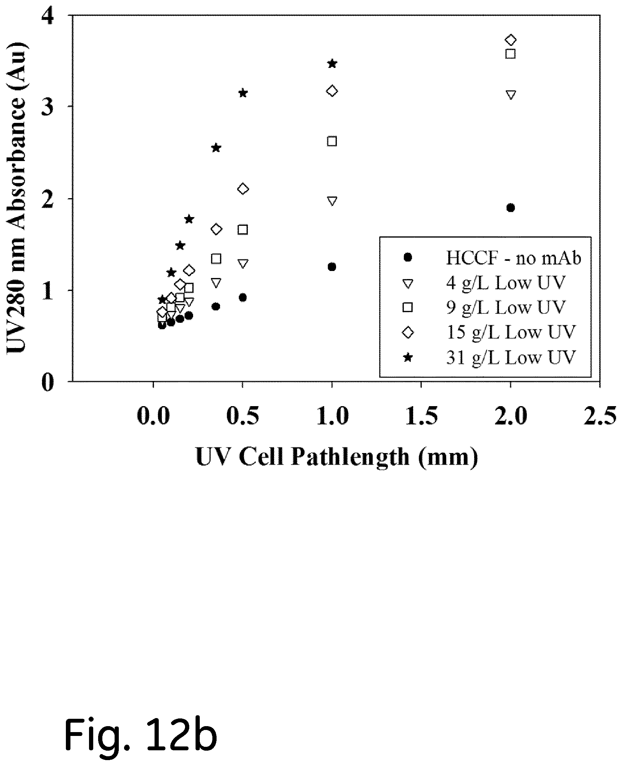

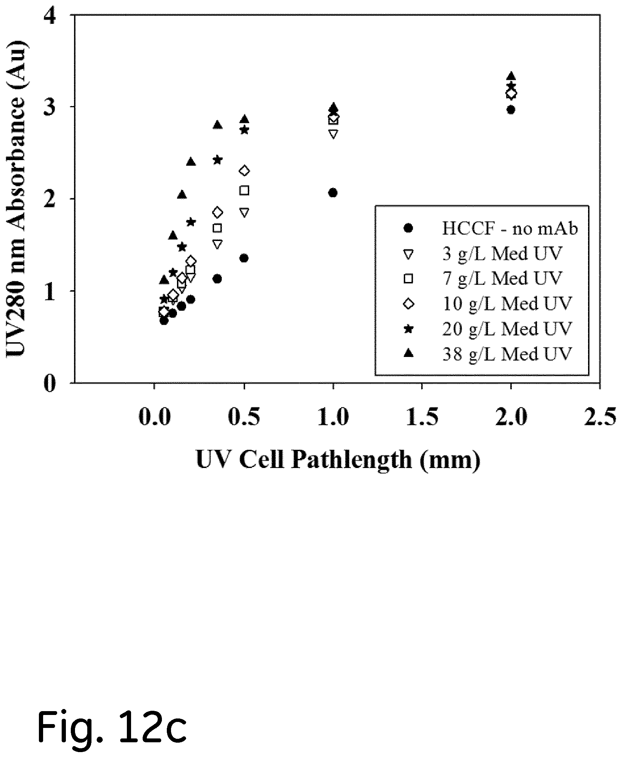

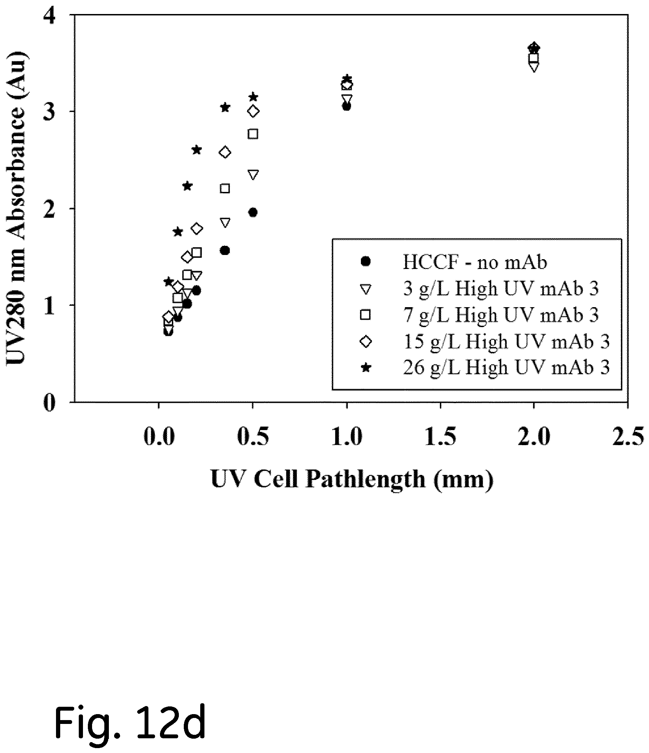

Figures 12A-12D show a comparison of UV280 nm absorbance versus UV cell pathlength. Figure 12A: mAb #1 high UV absorbance HCCF at titers ranging from 3 to 26 g/L. Figure 12B: mAb #1 low UV absorbance HCCF at titers ranging from 4 to 31 g/L. Figure 12C: mAb #2 medium UV absorbance HCCF at titers ranging from 3 to 38 g/L. Figure 12D: mAb #3 high UV absorbance HCCF at titers ranging from 3 to 26 g/L.

The UV280 nm absorbance of each HCCF sample including a sample without antibody (i.e. Protein A flowthrough) was measured for pathlengths ranging from 0.05 to 2 mm. For both high UV absorbing feeds (mAb #1 and mAb #3), the absorbance of the sample without antibody loses linearity at a 1 mm pathlength (as seen in the previous study). As the UV cell pathlength decreases, the absorbance of the blank sample becomes linear as a function of UV cell pathlength but there is a minimal ΔUV difference at pathlengths from 0.05 to 0.15 mm since the antibody absorbance significantly decreases as indicated in Figures 12A and 12D. The optimal pathlength range for HCCF containing high UV absorbance is between 0.2 to 0.5 mm for feeds ranging from about 3 to 30 g/L. At 0.5 mm pathlength, the linearity is beginning to decrease at higher concentrations of about 30 g/L. The linearity is maintained below the 0.5 mm pathlength, which indicates a pathlength between 0.2 to 0.35 mm would be best for maintaining UV linearity and ΔUV control under varying conditions. The minimum titer to maintain an appropriate ΔUV difference with respect to baseline drift and signal noise (i.e. at least~150 mAu) is about 3 g/L for the high UV HCCF feeds.

For the mAb #1 HCCF sample containing the lowest total UV absorption, the ΔUV difference is more pronounced even at lower pathlengths (down to 0.35 mm) if target protein exceeds 4 g/L as indicated in Figure 12B. The optimal pathlength for dynamical control is 0.35 mm for titers covering the range from about 3 to 31g/L. Even for conditions with low HCCF background ΔUV can be determined. For the mAb #2 HCCF sample containing the medium UV absorption, the optimal UV cell pathlengths for dynamic control are between 0.2 to 0.5 mm, as indicated in Figure 12C. However, at the highest titer condition of 26 g/L, the UV absorbance loses linearity at the 0.5 mm pathlength but is marginally acceptable at the 0.35 mm pathlength. Based on analysis of the different HCCF feeds, the best compromise is 0.35 mm pathlength to achieve a robust difference in ΔUV and also maintain linearity for HCCF titers from about 3 to 31g/L. The ΔUV becomes unstable with varying loading conditions. The ΔUV will change based on the ratio of product to impurities. For higher concentrations than 31g/L, either a UV cell with a shorter path length than 0.35 mm should be considered or an alternative UV cell wavelength where the analyte has a lower extinction coefficient while conserving the cell pathlength.

The ability to dynamically control protein loading using the ΔUV functionality for a 3C-PCC column system was tested using the four HCCF feeds listed in Table I. In order to challenge the ability of the UV280 nm UV cell to control protein loading, the ΔUV percentage was set at 70% for all of the runs. Performance metrics for the column experiments included a comparison of yield and purity levels to the control, which is a batch mode process to determine the success of the dynamic control function. Protein loading and UV280 nm difference between the UV pre and post column in the start-up phase and within the loop were monitored for each run to aid in determination of the ability to control protein loading using the UV280nm shorter pathlength cells.

This higher loading in the start-up phase could result in some antibody that is not captured by the second column in the loading zone and hence lost in the flowthrough as indicated in Table IV.

**Table IV:** Protein A chromatography yield, protein loading, and UV280 nm difference (mAu) using four different types of HCCF with the protein column loading controlled by a ΔUV setpoint of 70%.

| Cell Culture Feed | 3C-PCC | Protein Loading (g/L) | | UV 280: nm Difference (mAu) | |
|---|---|---|---|---|---|
| mAb 1 Low UV | Yield (%) | Start-up Phase | Within Loop | Start-up Phase | Within Loop |
| control | 93 | | | | |
| 4 g/L | 91 | 72 | 54 | 176 | 179 |
| 10 g/L | 89 | 73 | 56 | 368 | 355 |
| 15 g/L | 84 | 89 | 60 | 620 | 598 |
| 31g/L | 83 | 105 | 73 | 1137 | 1251 |
| mAb 1 High UV | Yield (%) | Start-up Phase | Within Loop | Start-up Phase | Within Loop |
| control | 95 | | | | |
| 3 g/L | 95 | 74 | 52 | 142 | 143 |
| 9 g/L | 94 | 78 | 55 | 306 | 294 |
| 19 g/L | 92 | 88 | 60 | 698 | 665 |
| 26 g/L | 91 | 93 | 58 | 865 | 780 |
| mAb 2 Med UV | Yield (%) | Start-up Phase | Within Loop | Start-up Phase | Within Loop |
| control | 95 | | | | |
| 4 g/L | 92 | 71 | 54 | 143 | 136 |
| 7 g/L | 91 | 80 | 59 | 288 | 299 |
| 10 g/L | 88 | 83 | 64 | 402 | 386 |
| 20 g/L | 87 | 103 | 68 | 704 | 684 |
| 41 g/L | 82 | 124 | 71 | 717 | 637 |
| mAb 3 High UV | Yield (%) | Start-up Phase | Within Loop | Start-up Phase | Within Loop |
| control | 97 | | | | |
| 3 g/L | 100 | 54 | 38 | 161 | 162 |
| 8 g/L | 99 | 71 | 48 | 345 | 340 |
| 15 g/L | 98 | 80 | 51 | 746 | 698 |
| 28 g/L | 95 | 96 | 51 | 1003 | 779 |

However, either a lower setpoint for ΔUV or possibly setting the baseline value earlier during the loading will decrease the protein loading and should increase the antibody recovery to within a few percentage of the batch mode control levels. The 41 g/L medium UV absorbance HCCF feed may be the only exception since the UV capabilities of this feed was shown to be outside of the linear range described in connection with figures 12A-12D. In addition, the difference in UV280nm signal is about 13 mAu outside of the loop and about 15 mAu inside of the loop for the 41 g/L feed compared to the 20 g/L feed, which also indicates a loss in UV cell linearity at the 41 g/L feed concentration. For feeds with at least about 35 g/L concentration, a UV cell with even shorter pathlength should be considered or a UV cell with a 300 nm wavelength.

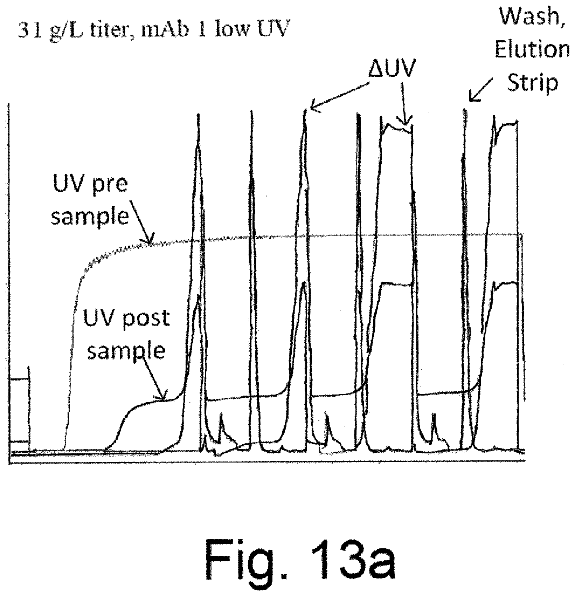

The ability of the UV pre and post sample cells to consistently control the ΔUV setpoint is shown in Figures 13A-13D, which illustrate examples of ÄKTA chromatographs showing protein loading control by ΔUV for mAb #1 low UV feed with HCCF titers ranging from 4 to 31 g/L. The UV280nm absorbance of the HCCF (i.e. UV sample pre) is shown in as UV pre sample while the UV post sample shows the UV280nm absorbance of the outlet of the first column in the loading zone (i.e. UV sample post). The periodic peaks show the wash, elution, and strip peaks for each of the three columns.

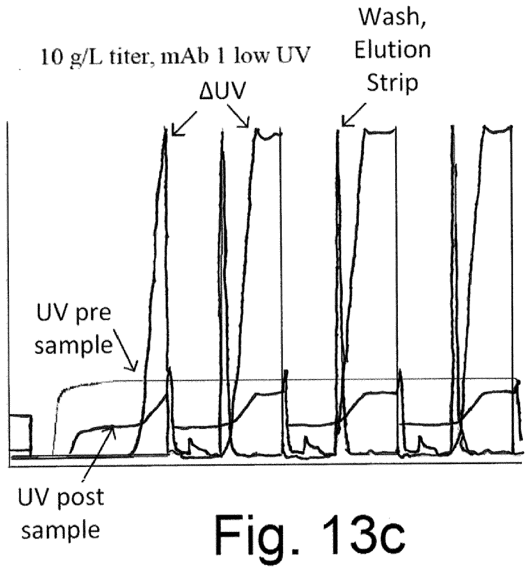

For HCCF titers up to 10 g/L, the column loading is consistent across each column as shown by the ÄKTA chromatograph, see Figures 13C-13D. However, when the HCCF titers increase up to 31 g/L, each column experiences a time difference in antibody breakthrough, which can be seen by the ΔUV function stopping the column loading at a setpoint of 70% during the loop at slightly different times for each column, see Figures 13A-13B. This result highlights the capability of dynamic control to maintain a consistent protein loading for each column, which is independent of the feedstock being purified.

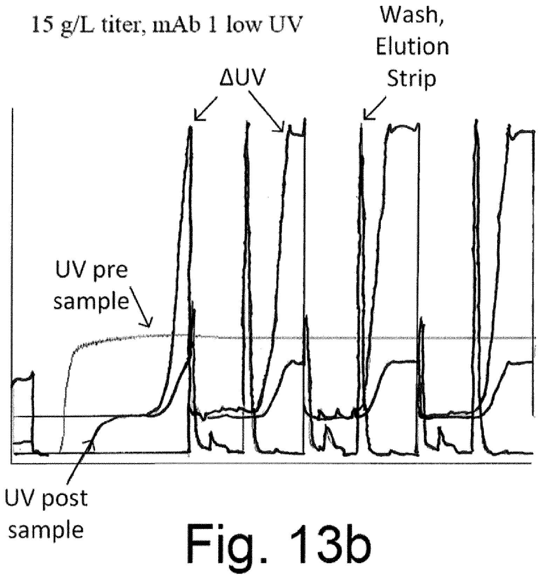

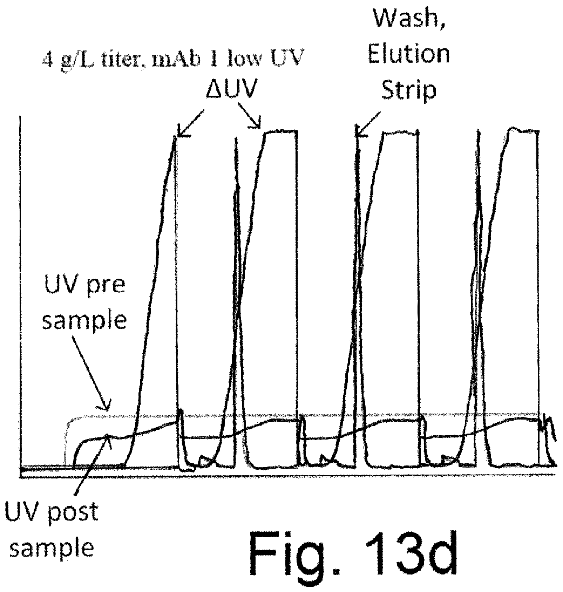

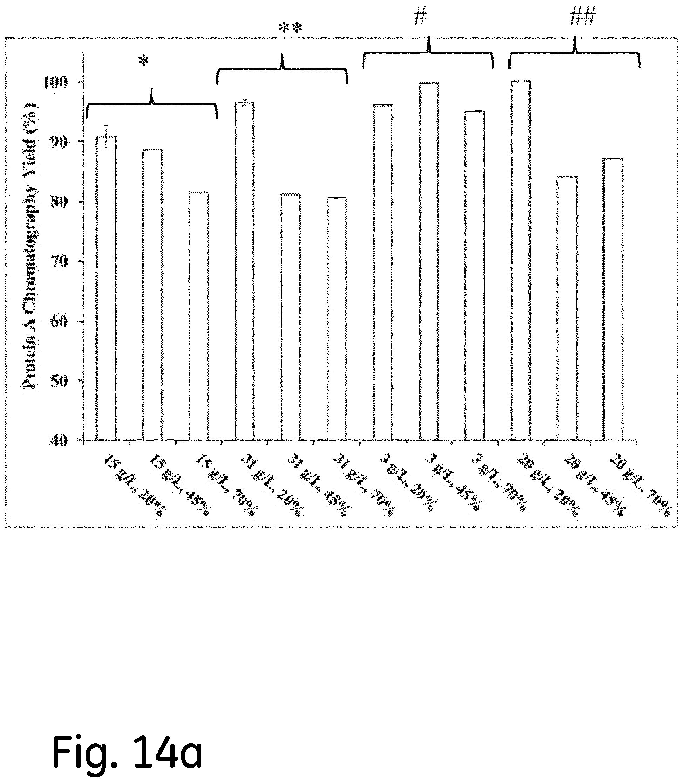

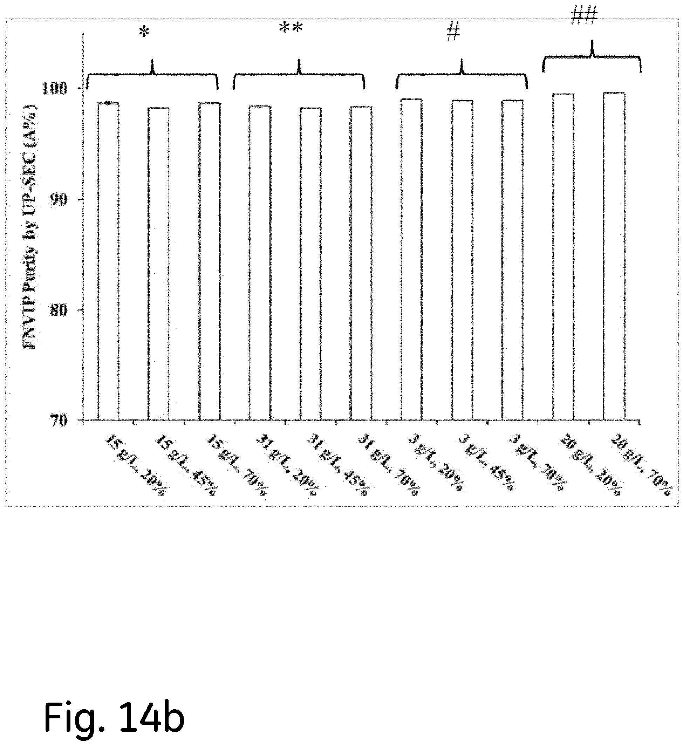

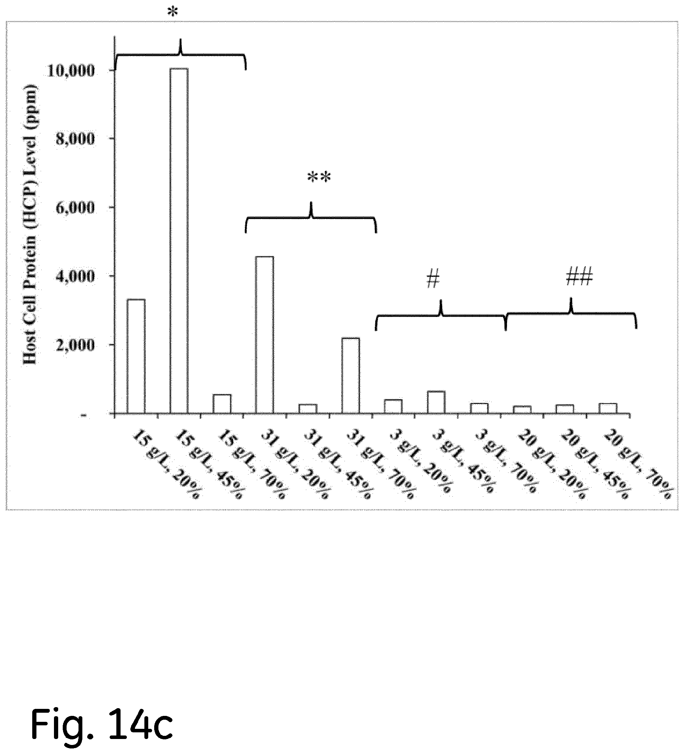

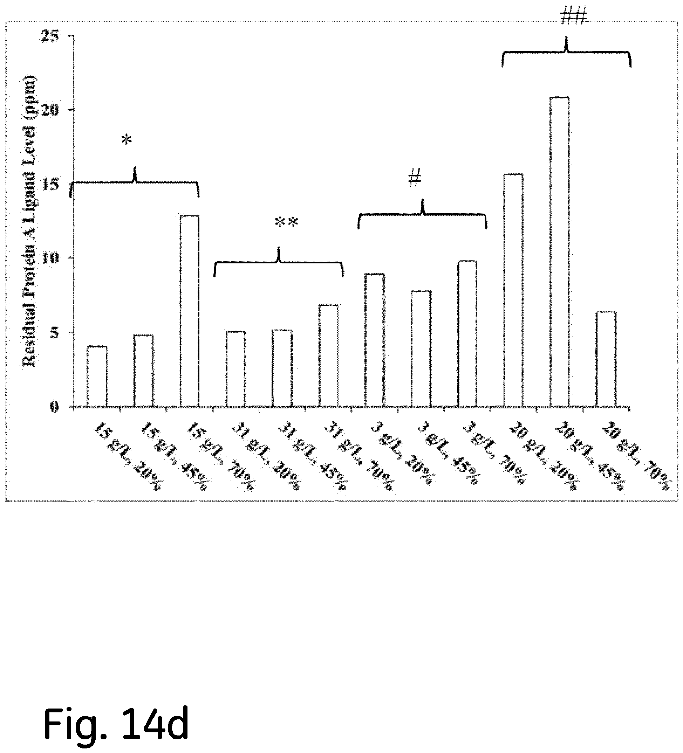

Additional studies were conducted to determine the impact of the ΔUV setpoint on the process performance of the Protein A chromatography step and quality of the FNVIP pools. The lower range of the ΔUV was set at 20% based on the DBC curves in connection with figures 13 and 14 in order to maintain a column loading in the loop that is similar to the lower concentration HCCF feeds. An additional ΔUV setpoint of 45% was also chosen in the middle of the range between 20 - 70% for comparison. HCCF feeds used for experimentation consisted of titers of at least 15 g/L with a low to medium UV background since yield loss has a stronger correlation with both of these parameters. A HCCF feed at 3 g/L was chosen for experimentation to confirm minimal impact of ΔUV within 20-70% on yield loss.

Figure 14A-14D show yield and quality data for FNVIP pools at the additional ΔUV setpoints. In figure 14A, Protein A chromatography yield shows a decline of about 10-20% as the ΔUV increases for all feeds studied except for the mAb #1 high UV feed. In figure 14B, purity by UP-SEC of the FNVIP pools is very consistent for each group of samples and does not change with higher protein loading (i.e. increase in ΔUV setpoint). In figure 14C, residual HCP levels were similar across the different ΔUV setpoints for the mAb #1 high UV, 3 g/L feed and mAb #2 medium UV, 20 g/L feed. HCP levels slightly increased and reached a maximum for the other two feeds. In figure 14D, residual Protein A ligand levels increase by about 5-10 ppm as the protein loading increases for each feed except for the mAb #2 medium UV, 20 g/L feed. * represents mAb 1 low UV, 15 g/L; ** represents mAb 1 low UV, 31g/L; # represents mAb #1 high UV, 3 g/L; ## represents mAb #2 medium UV, 20 g/L.

Yield loss across the Protein A chromatography step decreased substantially by about 13-19% when the ΔUV increased from 20 to 45% for HCCF titers of ≥ 20 g/L, see Figure 14A. Upon further investigation, the protein loading outside and within the loop is about 10-15 g/L higher at a ΔUV of 45%, which indicates possible antibody breakthrough on the second column in the loading zone as indicated in Table V. When the HCCF titer decreases to 15 g/L, yield loss is less pronounced and only about 2%, which is understandable given a similar column loading at both 20% and 45% ΔUV setpoints, see Figure 14A, Table V. The Protein A step yield is consistent for the 3 g/L feed and is maintained between about 95-100% regardless of an increase in ΔUV or column loading (Figure 14A, Table V). One hypothesis for the sensitivity of high titer HCCF feeds to ΔUV setpoint could be the slope of the actual antibody breakthrough is much steeper than the prediction given by the pore diffusion model. This could lead to underestimation of the antibody breakthrough and hence yield loss across the second column in the loading zone. In addition, the yield loss could also be larger due to the higher titer multiplied by the breakthrough volume will result in an increased amount of antibody lost in the flowthrough compared to lower titer HCCF feeds.

**Table V:** Protein A chromatography yield and protein loading on the column outside and inside of the loop using three different types of HCCF feeds with the column loading controlled by a ∆UV setpoint range of 20 - 70%.

| Feed | Sample | Yield (%) | Protein Loading (g/L) | |
| --- | --- | --- | --- | --- |
| | | | Outside of Loop | Within Loop |
| mab 1, low UV | 15 g/L, 20% | 88 | 84 | 53 |
| | | 90 | 81 | 53 |
| | | 94 | 80 | 53 |
| | 15 g/L, 45% | 89 | 84 | 53 |
| | 15 g/L, 70% | 79 | 94 | 62 |
| | | 84 | 89 | 60 |
| | 31 g/L, 20% | 96 | 74 | 51 |
| | | 97 | 84 | 51 |
| | | 96 | 77 | 51 |
| | 31 g/L, 45% | 81 | 90 | 58 |
| | 31 g/L, 70% | 78 | 105 | 72 |
| | | 83 | 105 | 73 |
| mab 1, high UV | 3 g/L, 70% | 95 | 74 | 52 |
| | 3 g/L, 45% | 100 | 57 | 44 |
| | 3 g/L, 20% | 96 | 47 | 38 |
| mAb 2, medium UV | 20 g/L, 20% | 100 | 93 | δ3 |
| | 20 g/L, 45% | 84 | 106 | 62 |
| | 20 g/L, 70% | 87 | 103 | 68 |

The UP-SEC purity of each FNVIP pool was consistent and independent of ∆UV range from 20-70%, see Figure 14B. The HCP levels were consistent for both 3 g/L and 20 g/L feeds but seemed to vary by up to about 9,000 ppm for the 15 and 31 g/L mAb 1, low UV feeds, see Figure 14C. The residual Protein A ligand increased slightly by about 3-10 ppm at a ∆UV equal to 70% for the 15 and 31 g/L mAb 1, low UV feeds, see Figure 14D. The residual Protein A ligand levels were similar for the 3 g/L feed regardless of ∆UV level, see Figure 14D. For the 20 g/L feed, the residual Protein A ligand level varied from 5-20 ppm for ∆UV range of 20-70%. The change in ∆UV setpoint did not have a large impact on product quality of the FNVIP pools for HCCF titers up to 31 g/L and was independent of cell culture UV280 background absorbance.

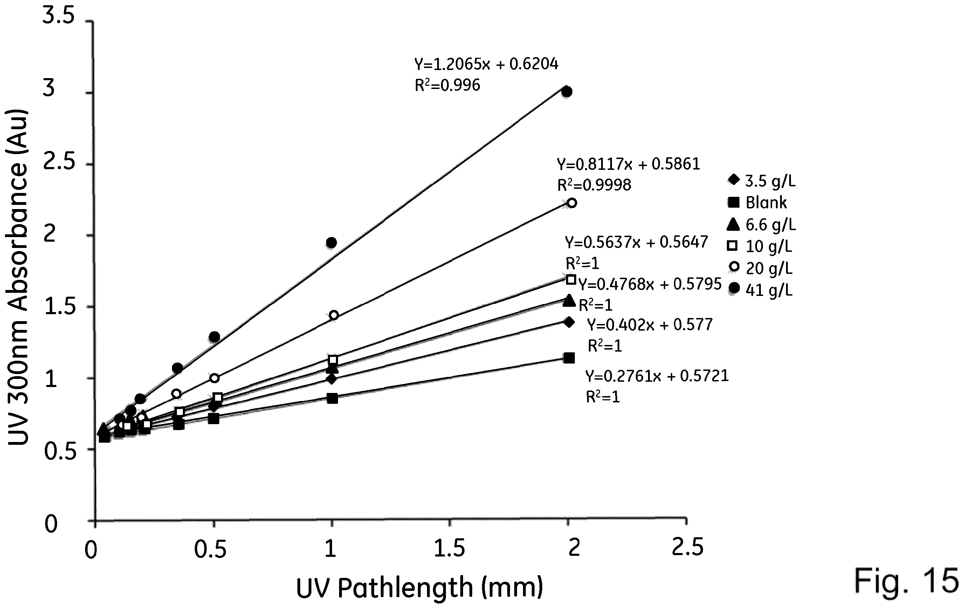

Figure 15 illustrates UV 300nm absorbance as a function of UV pathlength in mm. The relationship between linearity of the UV signal and different pathlengths for selected monoclonal antibody concentration in the feed material is shown.

It should be mentioned that the process of dynamic loading can be applied to fed-batch, intensified, and perfusion antibody cell culture streams. Basically, the dynamic control is feed independent. Furthermore, the claimed methods and systems may also be expanded to include non-antibody modalities and also cover resin modalities, including but not limited to Protein A resin, anion exchange, mixed mode, and HIC. Example of Protein A resins include, but are not limited to, Mabselect, Mabselect Sure, Mabselect Sure PCC, Mabselect Sure LX, Mabselect Xtra, and TOYOPEARL AF-rProtein A HC -650F.

Abbreviations

PCC: Periodic counter-current chromatography
3C-PCC: Three column periodic counter-current chromatography
UV: Ultraviolet

DBC: Dynamic binding capacity
RP-HPLC: Reverse phase high performance liquid chromatography
HCCF: Harvested cell culture fluid
UP-SEC: Ultra performance size exclusion chromatography
Med: Medium
mAb: Monoclonal antibody
HCP: Host cell protein
SMB: Simulated moving bed chromatography
SMCC: Sequential multi-column chromatography
HP-IEX: High performance ion exchange chromatography
FNVIP: Filtered neutralized viral inactivated Protein A product

REFERENCES

1. Anderson, N.G., Practical Use of Continuous Processing in Developing and Scaling Up Laboratory Processes. Org. Process Res. Dev., 2001. 5(6): p. 613-621.

2. Laird, T., Continuous Processes in Small-Scale Manufacture. Org. Process Res. Dev., 2007. 11(6): p. 927.

3. Masak, J., Ganapathy Subramanian (Ed.): Continuous Processing in Pharmaceutical Manufacturing. Chem. Listy. 109(6): p. 475.

4. Allison, G., Regulatory and Quality Considerations for Continuous Manufacturing. Presentation at the International Symposium on Continuous Manufacturing of Pharmaceuticals: Implementation, Technology, & Regulatory (Cambridge, MA), 2014.

5. Chatterjee, S., FDA Perspective on Continuous Manufacturing. Presentation at the IFPAC Annual Meeting (Baltimore, MD), 2012.

6. Hernandez, R., Continuous Manufacturing: A Changing Processing Paradigm. BioPharm International, 2015. 28(4): p. 20-27, 41.

7. Godawat, R., et al., End-to-end integrated fully continuous production of recombinant monoclonal antibodies. J Biotechnol. 213: p. 13-9.

8. Li, L., et al., A single-use, scalable perfusion bioreactor system. BioProcess Int., 2009. 7(6): p. 46, 48, 50, 52-54.

9. Voisard, D., et al., Potential of cell retention techniques for large-scale high-density perfusion culture of suspended mammalian cells. Biotechnol. Bioeng., 2003. 82(7): p. 751-765.

10. Walther, J., et al. Multi-staged experimental design for efficient process development of a continuous perfusion cell culture: American Chemical Society.

11. Angarita, M., et al., Twin-column CaptureSMB: a novel cyclic process for protein A affinity chromatography. J Chromatogr A. 1389: p. 85-95.

12. Godawat, R., et al., Periodic counter-current chromatography - design and operational considerations for integrated and continuous purification of proteins. Biotechnol. J. 7(12): p. 1496-1508.

13. Lacki, K.M. and L.M. Bryntesson. Protein A periodic counter-current chromatography for continuous antibody purification. 2004: American Chemical Society.

14. Ng, C.K.S., et al., Design of high productivity sequential multi-column chromatography for antibody capture. Food Bioprod. Process. 92(2): p. 233-241.

15. Trilisky, E., et al. Capacity assessment: DBC is not the whole story - implications for resin lifetime and sequential multicolumn chromatography: American Chemical Society.

16. Blom, H., et al. The use of dynamic control in periodic counter current chromatography: American Chemical Society.

17. Pollock, J., et al., Optimising the design and operation of semi-continuous affinity chromatography for clinical and commercial manufacture. J Chromatogr A. 1284: p. 17-27.

18. Warikoo, V., et al., Integrated continuous production of recombinant therapeutic proteins. Biotechnol Bioeng. 109(12): p. 3018-29.

19. Cooper, R.S. and D.A. Liberman, Fixed-bed adsorption kinetics with pore diffusion control. Ind. Eng. Chem., Fundam., 1970. 9(4): p. 620-3.

**Claims**

1. A method for determining operational status of a chromatography column (1; 39, 47, 59; 107, 109, 111, 113), comprising:

   - detecting a feed signal (21; 201) representative of the composition of a feed material provided to the inlet of the column by detecting the UV absorbance in the feed material,
   - detecting an effluent signal (23; 203, 205, 207, 209) representative of the composition of the effluent from the column;
   - using the feed signal and the effluent signal to determine operational status of the column, wherein the feed signal is generated using a first UV detector having a first UV cell pathlength operating at a first UV wavelength and the effluent signal is generated using a second UV detector having a second UV cell pathlength operating at a second UV wavelength, the method being **characterized in that** it comprises:
   - determining a first threshold value based on the detected UV absorbance in the feed material, and
   - selecting the first UV cell pathlength and/or first wavelength based on the first threshold value.

2. The method according to claim 1, **characterised by** continuously determining the operational status of the column (1; 39, 47, 59; 107, 109, 111, 113) during the chromatography process.

3. The method according to any one of the preceding claims, further comprising

   - using the feed signal (21; 201) and the effluent signal (23; 203, 205, 207, 209) to determine a deltasignal being the feed signal minus the effluent signal and a deltasignalmax (27) being the feed signal minus the effluent signal when the effluent signal shows a plateau (25) due to the fact that substantially all non-binding components have passed the column; and optionally
   - compensating for a time delay when determining the deltasignal and the deltasignalmax, said time delay representing the time it takes for a non-binding component to pass the column; and/or using the deltasignal to determine a breakthrough point (c) and/or a saturation point (d) of the column, said breakthrough point and saturation point being calculated as a respective certain predefined percentage of the deltasignalmax (27).

4. The method according to any one of the preceding claims, comprising detecting an effluent signal (203, 205, 207, 209) from each chromatography column (39, 47, 59; 107, 109, 111, 113) in a periodic counter current, PCC, system and using these effluent signals together with the feed signal (201) to continuously determine operational status of the different chromatography columns (39, 47, 59; 107, 109, 111, 113) of the PCC system during the chromatography process.

5. The method according to any of the preceding claims, wherein the feed material comprises:

   - impurities, resulting in background absorbance when measured in the first UV detector, and
   - product, resulting in an absorbance level of the product in the feed material when measured in the first UV detector,

   wherein the absorbance level of the product is at least forty percent of the background absorbance of the feed material, and wherein optionally the absorbance level of the product in the feed material is higher than the background absorbance, such as wherein the absorbance level of the product in the feed material is 70%-90% of the absorbance measured in the first UV detector.

6. The method according to any of the preceding claims, wherein the step of selecting the first UV cell pathlength comprises:

- selecting a fixed UV cell pathlength from a plurality of pre-selected UV cell pathlengths, or
- adjusting the UV cell pathlength,

such as wherein the step of selecting the first UV cell pathlength is performed until the first UV detector is within a linear range of detection.

7. The method according to any one of the preceding claims, wherein the step of selecting the first UV wavelength comprises adjusting the first UV wavelength, such as wherein the step of selecting the first UV wavelength is performed until the first UV detector is within a linear range of detection.

8. The method according to any of the preceding claims, wherein the step of determining the first threshold value comprises:

- estimating maximum UV absorbance in feed material, and
- selecting the first threshold value to ensure that the estimated maximum UV absorbance in the feed material is detectable in the first UV detector, wherein the method optionally further comprises calculating UV absorbance of the feed material, and the step of estimating maximum UV absorbance is based on the calculated UV absorbance.

9. The method according to claim 8, wherein the method further comprises measuring the UV absorbance of the feed material, and the step of estimating maximum UV absorbance is based on the measured UV absorbance, such as wherein the method further comprises continuously measuring the UV absorbance of the feed material, and selecting the first threshold value based on changes in UV absorbance.

10. The method according to any one of the preceding claims, comprising detecting the UV absorbance in the effluent, wherein the method further comprising:

- determining a second threshold value based on the detected UV absorbance in the effluent and/or the first threshold value, such as wherein the second threshold value is lower or equal to the first threshold value, and
- selecting the second UV cell pathlength and/or second UV wavelength based on the second threshold value,

wherein optionally the step of determining the second threshold value comprises:

- estimating UV absorbance for a selected breakthrough point in the effluent, and
- selecting the second threshold value to ensure that the estimated UV absorbance for the selected breakthrough point is detectable in the second UV detector.

11. The method according to claim 10, wherein:

a) the method further comprises measuring the UV absorbance in the effluent, and the step of estimating UV absorbance for the selected breakthrough point is based on the measured UV absorbance;
b) the method further comprises selecting the second UV cell pathlength to be equal to the first UV cell pathlength;
c) the step of selecting the second UV cell pathlength comprises:

- selecting a fixed UV cell pathlength from a plurality of pre-selected UV cell pathlengths, or
- adjusting the UV cell pathlength;

d) the step of selecting the second UV wavelength comprises adjusting the second UV wavelength; and/or
e) the step of selecting the second UV cell pathlength and/or second UV wavelength is performed until the second UV detector is within a linear range of detection when steps c) and/or d) are also carried out.

**12.** A method for controlling a chromatography system comprising at least one column (1; 39, 47, 59; 107, 109, 111, 113), comprising the steps of:

- determining operational status of the at least one chromatography column according to any one of the claims 1-11;
- controlling the start and stop of the different chromatography process steps according to the determined operational status; and
- optionally continuously determining the operational status during the chromatography process and in real time controlling the start and stop of the different chromatography process steps according to the determined operational status.

**13.** A method for controlling a periodic counter current chromatography system comprising at least two columns (39, 47, 59; 107, 109, 111, 113), comprising the steps of:

- detecting a feed signal (201) representative of the composition of a feed material provided to the inlet of the columns;
- detecting effluent signals (203, 205, 207, 209) representative of the composition of the effluent from each column in the system;
- determining the operational status of each chromatography column according to any one of the claims 1-11;
- controlling the feed to and between the columns in dependence of the determined operational status.

**14.** The method according to claim 13, comprising:

a) continuously determining the operational status during the chromatography process and in real time controlling the feed to and between the columns in dependence of the determined operational status;
b) controlling the flow rates for the feed and buffer pumps (31, 37; 101, 103, 105) in dependence of the determined operational status; and/or
c) compensating for any differences in the different column properties and/or flow rates by adjusting for how long, and in which position, different columns should be in the loading zone according to the determined operational status.

**15.** A chromatography system comprising at least one chromatography column (1; 39, 47, 59; 107, 109, 111, 113), a detector for detecting a feed signal (21; 201) representative of the composition of a feed material provided to the inlet of the column, a UV absorbance detector for detecting the UV absorbance in the feed material and a detector for detecting an effluent signal (23; 203, 205, 207, 209) representative of the composition of the effluent from the column, said chromatography system being **characterized in that** it is configured to :

- determine operational status of the at least one chromatography column according to any one of the claims 1-11;
- control the start and stop of the different chromatography process steps according to the determined operational status; and
- optionally further configured to:
continuously determine the operational status during the chromatography process, and in real time control the start and stop of the different chromatography process steps according to the determined operational status.

**16.** A periodic counter current chromatography system comprising at least two columns (39, 47, 59; 107, 109, 111, 113), the periodic current chromatography system is configured to:

- detect a feed signal (201) representative of the composition of a feed material provided to the inlet of the columns;
- detect effluent signals (203, 205, 207, 209) representative of the composition of the effluent from each column in the system;
- determine the operational status of each chromatography column according to any one of the claims 1-11; and
- control the feed to and between the columns in dependence of the determined operational status.

**17.** The periodic counter current chromatography system according to claim 16, further configured to:

a) continuously determine the operational status during the chromatography process and in real time control the feed to and between the columns in dependence of the determined operational status;
b) control the flow rates for the feed and buffer pumps (31, 37; 101, 103, 105) in dependence of the determined operational status; and/or

c) compensate for any differences in the different column properties and/or flow rates by adjusting for how long, and in which position, different columns should be in the loading zone according to the determined operational status.

**Patentansprüche**

1. Verfahren zum Bestimmen eines Betriebsstatus einer Chromatographie-Säule (1; 39, 47, 59; 107, 109, 111, 113), umfassend:

   - Erfassen eines Einspeisesignals (21; 201), das repräsentativ ist für die Zusammensetzung eines Einspeisematerials, das an dem Eingang der Säule bereitgestellt wird durch Erfassen der UV-Absorbanz im Einspeisematerial,
   - Erfassen eines Eluatsignals (23; 203, 205, 207, 209), das repräsentativ ist für die Zusammensetzung des Eluats aus der Säule;
   - Verwenden des Einspeisesignals und des Eluatsignals, um einen Betriebsstatus der Säule zu bestimmen, wobei das Einspeisesignal unter Verwendung eines ersten UV-Detektors erzeugt wird, der eine erste UV-Zellenweglänge aufweist, der bei einer ersten UV-Wellenlänge betrieben wird, und das Eluatsignal unter Verwendung eines zweiten UV-Detektors erzeugt wird, der eine zweite UV-Zellenweglänge aufweist, der bei einer zweiten UV-Wellenlänge betrieben wird, wobei das Verfahren **dadurch gekennzeichnet ist, dass** es umfasst:
   - Bestimmen eines ersten Schwellenwerts basierend auf der erfassten UV-Absorbanz im Einspeisematerial, und
   - Auswählen der ersten UV-Zellenweglänge und/oder ersten Wellenlänge basierend auf dem ersten Schwellenwert.

2. Verfahren nach Anspruch 1, **gekennzeichnet durch** kontinuierliches Bestimmen des Betriebsstatus der Säule (1; 39, 47, 59; 107, 109, 111, 113) während des Chromatographie-Prozesses.

3. Verfahren nach einem der vorstehenden Ansprüche, weiter umfassend

   - Verwenden des Einspeisesignals (21; 201) und des Eluatsignals (23; 203, 205, 207, 209), um ein Deltasignal zu bestimmen, das das Einspeisesignal minus das Eluatsignal ist, und ein Deltasignalmax (27), das das Einspeisesignal minus das Eluatsignal ist, wenn das Eluatsignal ein Plateau (25) aufgrund der Tatsache aufzeigt, dass im Wesentlichen alle nichtbindenden Komponenten die Säule durchquert haben; und wahlweise
   - Ausgleichen einer Zeitverzögerung bei der Bestimmung des Deltasignals und des Deltasignalmax, wobei die Zeitverzögerung die Zeit darstellt, die eine nichtbindende Komponente zum Durchqueren der Säule benötigt; und/oder Verwenden des Deltasignals, um einen Durchbruchspunkt (c) und/oder einen Sättigungspunkt (d) der Säule zu bestimmen, wobei der Durchbruchspunkt und Sättigungspunkt als ein jeweiliger gewisser vorab definierter Prozentsatz des Deltasignalmax (27) berechnet werden.

4. Verfahren nach einem der vorstehenden Ansprüche, umfassend Erfassen eines Eluatsignals (203, 205, 207, 209) von jeder Chromatographie-Säule (39, 47, 59; 107,109, 111, 113) in einem System mit periodischem Gegenstrom, PCC, und Verwenden dieser Eluatsignale zusammen mit dem Einspeisesignal (201), um einen Betriebsstatus der unterschiedlichen Chromatographie-Säulen (39, 47, 59; 107, 109, 111, 113) des PCC-Systems während des Chromatographie-Prozesses kontinuierlich zu bestimmen.

5. Verfahren nach einem der vorstehenden Ansprüche, wobei das Einspeisematerial umfasst:

   - Verunreinigungen, die bei Messung im ersten UV-Detektor zu Hintergrundabsorbanz führen, und
   - Produkt, das bei Messung im ersten UV-Detektor zu einem Absorbanzniveau des Produkts im Einspeisematerial führt,

   wobei das Absorbanzniveau des Produkts mindestens vierzig Prozent der Hintergrundabsorbanz des Einspeisematerials ist, und wobei das Absorbanzniveau des Produkts im Einspeisematerial wahlweise höher ist als die Hintergrundabsorbanz, wie etwa wobei das Absorbanzniveau des Produkts im Einspeisematerial 70% bis 90% der im ersten UV-Detektor gemessenen Absorbanz beträgt.

6. Verfahren nach einem der vorstehenden Ansprüche, wobei der Schritt des Auswählens der ersten UV-Zellenweglänge umfasst:

- Auswählen einer festen UV-Zellenweglänge aus einer Vielzahl von vorab ausgewählten UV-Zellenweglängen, oder
- Einstellen der UV-Zellenweglänge,

wie etwa wobei der Schritt des Auswählens der ersten UV-Zellenweglänge durchgeführt wird, bis sich der erste UV-Detektor innerhalb eines linearen Erfassungsbereichs befindet.

7.   Verfahren nach einem der vorstehenden Ansprüche, wobei der Schritt des Auswählens der ersten UV-Wellenlänge umfasst, die erste UV-Wellenlänge einzustellen, wie etwa wobei der Schritt des Auswählens der ersten UV-Wellenlänge durchgeführt wird, bis sich der erste UV-Detektor innerhalb eines linearen Erfassungsbereichs befindet.

8.   Verfahren nach einem der vorstehenden Ansprüche, wobei der Schritt des Bestimmens des ersten Schwellenwerts umfasst:

- Schätzen der maximalen UV-Absorbanz im Einspeisematerial, und
- Auswählen des ersten Schwellenwerts, um sicherzustellen, dass die geschätzte maximale UV-Absorbanz im Einspeisematerial im ersten UV-Detektor erfassbar ist, wobei das Verfahren wahlweise weiter umfasst, die UV-Absorbanz des Einspeisematerials zu berechnen, und der Schritt des Schätzens der maximalen UV-Absorbanz auf der berechneten UV-Absorbanz basiert.

9.   Verfahren nach Anspruch 8, wobei das Verfahren weiter umfasst, die UV-Absorbanz des Einspeisematerials zu messen, und der Schritt des Schätzens der maximalen UV-Absorbanz auf der gemessenen UV-Absorbanz basiert, wie etwa wobei das Verfahren weiter umfasst, die UV-Absorbanz des Einspeisematerials kontinuierlich zu messen und den ersten Schwellenwert basierend auf Änderungen der UV-Absorbanz auszuwählen.

10.  Verfahren nach einem der vorstehenden Ansprüche, umfassend Erfassen der UV-Absorbanz im Eluat, wobei das Verfahren weiter umfasst:

- Bestimmen eines zweiten Schwellenwerts basierend auf der erfassten UV-Absorbanz im Eluat und/oder dem ersten Schwellenwert, wie etwa wobei der zweite Schwellenwert kleiner oder gleich dem ersten Schwellenwert ist, und
- Auswählen der zweiten UV-Zellenweglänge und/oder zweiten UV-Wellenlänge basierend auf dem zweiten Schwellenwert,

wobei der Schritt des Bestimmens des zweiten Schwellenwerts wahlweise umfasst:

- Schätzen von UV-Absorbanz für einen ausgewählten Durchbruchspunkt im Eluat, und
- Auswählen des zweiten Schwellenwerts, um sicherzustellen, dass die geschätzte UV-Absorbanz für den ausgewählten Durchbruchspunkt im zweiten UV-Detektor erfassbar ist.

11.  Verfahren nach Anspruch 10, wobei:

a) das Verfahren weiter umfasst, die UV-Absorbanz im Eluat zu messen, und der Schritt des Schätzens der UV-Absorbanz für den ausgewählten Durchbruchspunkt auf der gemessenen UV-Absorbanz basiert;
b) das Verfahren weiter umfasst, die zweite UV-Zellenweglänge so auszuwählen, dass sie gleich der ersten UV-Zellenweglänge ist;
c) der Schritt des Auswählens der zweiten UV-Zellenweglänge umfasst:

- Auswählen einer festen UV-Zellenweglänge aus einer Vielzahl von vorab ausgewählten UV-Zellenweglängen, oder
- Einstellen der UV-Zellenweglänge;

d) der Schritt des Auswählens der zweiten UV-Wellenlänge umfasst, die zweite UV-Wellenlänge einzustellen; und/oder
e) der Schritt des Auswählens der zweiten UV-Zellenweglänge und/oder der zweiten UV-Wellenlänge durchgeführt wird, bis sich der zweite UV-Detektor innerhalb eines linearen Erfassungsbereichs befindet, wenn die Schritte c) und/oder d) ebenfalls ausgeführt werden.

12. Verfahren zum Steuern eines Chromatographie-Systems, das mindestens eine Säule (1; 39, 47, 59; 107, 109, 111, 113) umfasst, umfassend die folgenden Schritte:

- Bestimmen eines Betriebsstatus der mindestens einen Chromatographie-Säule nach einem der Ansprüche 1-11;
- Steuern des Startens und Stoppens der unterschiedlichen Chromatographie-Prozessschritte gemäß dem bestimmten Betriebsstatus; und
- wahlweise kontinuierliches Bestimmen des Betriebsstatus während des Chromatographie-Prozesses und in Echtzeit Steuern des Startens und Stoppens der unterschiedlichen Chromatographie-Prozessschritte gemäß dem bestimmten Betriebsstatus.

13. Verfahren zum Steuern eines Chromatographie-Systems mit periodischem Gegenstrom, das mindestens zwei Säulen (39, 47, 59; 107, 109, 111, 113) umfasst, umfassend die folgenden Schritte:

- Bestimmen eines Einspeisesignals (201), das repräsentativ ist für die Zusammensetzung des Einspeisematerials, das an dem Eingang der Säulen bereitgestellt wird;
- Erfassen von Eluatsignalen (23; 203, 205, 207, 209), die repräsentativ sind für die Zusammensetzung des Eluats aus jeder Säule im System;
- Bestimmen des Betriebsstatus jeder Chromatographie-Säule nach einem der Ansprüche 1-11;
- Steuern der Einspeisung zu und zwischen den Säulen in Abhängigkeit vom bestimmten Betriebsstatus.

14. Verfahren nach Anspruch 13, umfassend:

a) kontinuierliches Bestimmen des Betriebsstatus während des Chromatographie-Prozesses und in Echtzeit Steuern der Einspeisung zu und zwischen den Säulen in Abhängigkeit vom bestimmten Betriebsstatus;
b) Steuern der Flussraten für die Einspeisungs- und Pufferpumpen (31, 37; 101, 103, 105) in Abhängigkeit vom bestimmten Betriebsstatus; und/oder
c) Ausgleichen jeglicher Unterschiede in den unterschiedlichen Säulenmerkmalen und/oder Flussraten durch Einstellen, wie lange und in welcher Position unterschiedliche Säulen gemäß dem bestimmten Betriebsstatus im Ladebereich sein sollten.

15. Chromatographie-System, umfassend mindestens eine Chromatographie-Säule (1; 39, 47, 59; 107, 109, 111, 113), einen Detektor zum Erfassen eines Einspeisesignals (21; 201), das repräsentativ ist für die Zusammensetzung eines Einspeisematerials, das an dem Eingang der Säule bereitgestellt wird, einen UV-Absorbanzdetektor zum Erfassen der UV-Absorbanz im Einspeisematerial und einen Detektor zum Erfassen eines Eluatsignals (23; 203, 205, 207, 209), das repräsentativ ist für die Zusammensetzung des Eluates aus der Säule, wobei das Chromatographie-System **dadurch gekennzeichnet ist, dass** es zu Folgendem ausgelegt ist:

- Bestimmen eines Betriebsstatus der mindestens einen Chromatographie-Säule nach einem der Ansprüche 1-11;
- Steuern des Startens und Stoppens der unterschiedlichen Chromatographie-Prozessschritte gemäß dem bestimmten Betriebsstatus; und
- wahlweise weiter zu Folgendem ausgelegt:
kontinuierliches Bestimmen des Betriebsstatus während des Chromatographie-Prozesses und in Echtzeit Steuern des Startens und Stoppens der unterschiedlichen Chromatographie-Prozessschritte gemäß dem bestimmten Betriebsstatus.

16. Chromatographie-System mit periodischem Gegenstrom, das mindestens zwei Säulen (39, 47, 59; 107,109, 111, 113) umfasst, wobei das Chromatographie-System mit periodischem Strom zu Folgendem ausgelegt ist:

- Bestimmen eines Einspeisesignals (201), das repräsentativ ist für die Zusammensetzung des Einspeisematerials, das an dem Eingang der Säulen bereitgestellt wird;
- Erfassen von Eluatsignalen (23; 203, 205, 207, 209), die repräsentativ sind für die Zusammensetzung des Eluats aus jeder Säule im System;
- Bestimmen des Betriebsstatus jeder Chromatographie-Säule nach einem der Ansprüche 1 - 11; und
- Steuern der Einspeisung zu und zwischen den Säulen in Abhängigkeit vom bestimmten Betriebsstatus.

**17.** Chromatographie-System mit periodischem Gegenstrom nach Anspruch 16, das weiter zu Folgendem ausgelegt ist:

a) kontinuierliches Bestimmen des Betriebsstatus während des Chromatographie-Prozesses und in Echtzeit Steuern der Einspeisung zu und zwischen den Säulen in Abhängigkeit vom bestimmten Betriebsstatus;
b) Steuern der Flussraten für die Einspeisungs- und Pufferpumpen (31, 37; 101, 103, 105) in Abhängigkeit vom bestimmten Betriebsstatus; und/oder
c) Ausgleichen jeglicher Unterschiede in den unterschiedlichen Säulenmerkmalen und/oder Flussraten durch Einstellen, wie lange und in welcher Position unterschiedliche Säulen gemäß dem bestimmten Betriebsstatus im Ladebereich sein sollten.

**Revendications**

**1.** Procédé pour déterminer l'état de fonctionnement d'une colonne de chromatographie (1 ; 39, 47, 59 ; 107, 109, 111, 113), comprenant :

- la détection d'un signal d'alimentation (21 ; 201) représentatif de la composition d'un matériau d'alimentation présenté à l'entrée de la colonne par la détection d'absorbance UV dans le matériau d'alimentation,
- la détection d'un signal d'effluent (23 ; 203, 205, 207, 209) représentatif de la composition de l'effluent en provenance de ladite colonne ;
- l'utilisation du signal d'alimentation et du signal d'effluent pour déterminer l'état de fonctionnement de la colonne, dans lequel le signal d'alimentation est généré en utilisant un premier détecteur UV présentant une première longueur de trajet de cellule UV fonctionnant à une première longueur d'onde UV et le signal d'effluent est généré en utilisant un second détecteur UV présentant une seconde longueur de trajet de cellule UV fonctionnant à une seconde longueur d'onde UV, le procédé étant **caractérisé en ce qu'**il comprend les étapes consistant à :

- déterminer une première valeur seuil sur la base de l'absorbance UV détectée dans le matériau d'alimentation, et
- sélectionner la première longueur de trajet et/ou la première longueur d'onde de cellule UV sur la base de la première valeur seuil.

**2.** Procédé selon la revendication 1, **caractérisé par** la détermination en continu de l'état de fonctionnement de la colonne (1 ; 39, 47, 59 ; 107, 109, 111, 113) lors du processus de chromatographie.

**3.** Procédé selon l'une quelconque des revendications précédentes, comprenant en outre les étapes consistant à :

- utiliser le signal d'alimentation (21 ; 201) et le signal d'effluent (23 ; 203, 205, 207, 209) pour déterminer un signal delta qui est le signal d'alimentation moins le signal d'effluent et un signal delta max (27) qui est le signal d'alimentation moins le signal d'effluent lorsque le signal d'effluent présente un plateau (25) en raison du fait que sensiblement tous les composants non liants ont traversé la colonne ; et facultativement
- compenser un retard lors de la détermination du signal delta et du signal delta max, ledit retard représentant le temps nécessaire à un composant non liant pour passer la colonne ; et/ou utiliser le signal delta pour déterminer un point de percée (c) et/ou un point de saturation (d) de la colonne, ledit point de percée et point de saturation étant calculés comme un certain pourcentage prédéfini respectif du signal delta max (27).

**4.** Procédé selon l'une quelconque des revendications précédentes, comprenant la détection d'un signal d'effluent (203, 205, 207, 209) en provenance de chaque colonne de chromatographie (39, 47, 59 ; 107,109, 111, 113) dans un système à contre-courant périodique, PCC, et en utilisant ces signaux d'effluent conjointement avec le signal d'alimentation (201) pour déterminer en continu l'état de fonctionnement des différentes colonnes de chromatographie (39, 47, 59 ; 107, 109, 111, 113) du système PCC pendant le processus de chromatographie.

**5.** Procédé selon l'une quelconque des revendications précédentes, dans lequel le matériau d'alimentation comprend :

- des impuretés, qui entraînent une absorbance de fond lors de la mesure dans le premier détecteur UV, et
- un produit, qui entraîne un niveau d'absorbance du produit dans le matériau d'alimentation lorsqu'il est mesuré dans le premier détecteur UV,

dans lequel le niveau d'absorbance du produit est au moins égal à quarante pour cent de l'absorbance de fond du matériau d'alimentation, et dans lequel, facultativement, le niveau d'absorbance du produit dans le matériau d'alimentation est supérieur à l'absorbance de fond, par exemple dans lequel le niveau d'absorbance du produit dans le matériau d'alimentation est de 70 % à 90 % de l'absorbance mesurée dans le premier détecteur UV.

**6.** Procédé selon l'une quelconque des revendications précédentes, dans lequel l'étape consistant à sélectionner la première longueur de trajet de cellule UV comprend les étapes consistant à :

- sélectionner une longueur de trajet de cellule UV fixe parmi une pluralité de longueurs de trajet de cellule UV présélectionnées, ou
- ajuster la longueur de trajet de cellule UV,

tel que dans lequel l'étape consistant à sélectionner la première longueur de trajet de cellule UV est effectuée jusqu'à ce que le premier détecteur UV soit dans une gamme linéaire de détection.

**7.** Procédé selon l'une quelconque des revendications précédentes, dans lequel l'étape consistant à sélectionner la première longueur d'onde UV comprend l'ajustement de la première longueur d'onde UV, tel que dans lequel l'étape de sélection de la première longueur d'onde UV est effectuée jusqu'à ce que le premier détecteur UV soit dans une gamme linéaire de détection.

**8.** Procédé selon l'une quelconque des revendications précédentes, dans lequel l'étape de détermination de la première valeur seuil comprend les étapes consistant à :

- estimer l'absorbance UV maximale dans le matériau d'alimentation, et
- sélectionner la première valeur seuil pour garantir que l'absorbance UV maximale estimée dans le matériau d'alimentation est détectable dans le premier détecteur UV, dans lequel le procédé comprend en outre facultativement le calcul de l'absorbance UV du matériau d'alimentation, et l'étape d'estimation de l'absorbance UV maximale est basée sur l'absorbance UV calculée.

**9.** Procédé selon la revendication 8, dans lequel le procédé comprend en outre la mesure de l'absorbance UV du matériau d'alimentation, et l'étape d'estimation de l'absorbance UV maximale est basée sur l'absorbance UV mesurée, tel que dans lequel le procédé comprend en outre la mesure en continu de l'absorbance UV du matériau d'alimentation, et la sélection de la première valeur seuil basée sur les changements d'absorbance UV.

**10.** Procédé selon l'une quelconque des revendications précédentes, comprenant la détection de l'absorbance UV dans l'effluent, dans lequel le procédé comprend en outre les étapes consistant à :

- déterminer une seconde valeur seuil basée sur l'absorbance UV détectée dans l'effluent et/ou la première valeur seuil, tel que dans lequel la seconde valeur seuil est inférieure ou égale à la première valeur seuil, et
- sélectionner la seconde longueur de trajet et/ou la seconde longueur d'onde de cellule UV sur la base de la seconde valeur seuil.

dans lequel, facultativement, l'étape de détermination de la seconde valeur seuil comprend les étapes consistant à :

- estimer l'absorbance UV pour un point de percée sélectionné dans l'effluent, et
- sélectionner la seconde valeur seuil pour garantir que l'absorbance UV estimée pour le point de percée sélectionné est détectable dans le second détecteur UV.

**11.** Procédé selon la revendication 10, dans lequel :

a) le procédé comprend en outre la mesure de l'absorbance UV dans l'effluent, et l'étape d'estimation de l'absorbance UV pour le point de percée sélectionné est basée sur l'absorbance UV mesurée ;
b) le procédé comprend en outre la sélection de la seconde longueur de trajet de cellule UV pour qu'elle soit égale à la première longueur de trajet de cellule UV ;
c) l'étape de sélection de la seconde longueur de trajet de cellule UV comprend les étapes consistant à :

- sélectionner une longueur de trajet de cellule UV fixe parmi une pluralité de longueurs de trajet de cellule UV présélectionnées, ou

- ajuster la longueur de trajet de cellule UV ;

d) l'étape de sélection de la seconde longueur d'onde UV comprend l'ajustement de la seconde longueur d'onde UV ; et/ou

e) l'étape de sélection de la seconde longueur de trajet de cellule UV et/ou la seconde longueur d'onde UV est effectuée jusqu'à ce que le second détecteur UV se trouve dans une plage linéaire de détection lorsque les étapes c) et/ou d) sont également effectuées.

12. Procédé pour commander le système de chromatographie comprenant au moins une colonne (1 ; 39, 47, 59 ; 107, 109, 111, 113), comprenant les étapes consistant à :

- déterminer l'état de fonctionnement de la au moins une colonne de chromatographie selon l'une quelconque des revendications 1 à 11 ;
- commander le début et la fin des différentes étapes du processus de chromatographie selon l'état de fonctionnement déterminé ; et
- éventuellement déterminer en continu l'état de fonctionnement pendant le processus de chromatographie et commander en temps réel le début et la fin des différentes étapes du processus de chromatographie selon l'état de fonctionnement déterminé.

13. Procédé pour commander un système à contre-courant périodique de chromatographie comprenant au moins deux colonnes (39, 47, 59; 107, 109, 111, 113), comprenant les étapes consistant à :

- détecter un signal d'alimentation (201) représentatif de la composition d'un matériau d'alimentation présenté à l'entrée des colonnes ;
- détecter des signaux d'effluent (203, 205, 207, 209) représentatifs de la composition de l'effluent en provenance de chaque colonne dans le système ;
- déterminer l'état de fonctionnement de chaque colonne de chromatographie selon l'une quelconque des revendications 1-11 ;
- commander l'alimentation vers et entre les colonnes en fonction de l'état de fonctionnement déterminé.

14. Procédé selon la revendication 13, comprenant les étapes consistant à :

a) déterminer en continu l'état de fonctionnement pendant le processus de chromatographie et commander en temps réel l'alimentation vers et entre les colonnes en fonction de l'état de fonctionnement déterminé ;
b) commander les débits des pompes d'alimentation et des pompes tampons (31, 37 ; 101, 103, 105) en fonction de l'état de fonctionnement déterminé ; et/ou
c) compenser les différences entre les propriétés et/ou les débits dans les différentes colonnes en ajustant la durée et la position des différentes colonnes dans la zone de chargement en fonction de l'état de fonctionnement déterminé.

15. Système de chromatographie comprenant au moins un colonne de chromatographie (1 ; 39, 47, 59; 107, 109, 111, 113), un détecteur pour détecter un signal d'alimentation (21; 201) représentatif de la composition d'un matériau d'alimentation présenté à l'entrée de la colonne, un détecteur d'absorbance UV pour détecter l'absorbance UV dans la matériau d'alimentation et un détecteur pour détecter un signal d'effluent (23 ; 203, 205, 207, 209) représentatif de la composition de l'effluent en provenance de la colonne, ledit système de chromatographie étant **caractérisé en ce qu'**il est configuré pour :

- déterminer l'état de fonctionnement de la au moins une colonne de chromatographie selon l'une quelconque des revendications 1 à 11 ;
- commander le début et la fin des différentes étapes du processus de chromatographie selon l'état de fonctionnement déterminé ; et
- facultativement configuré en outre pour :
déterminer en continu l'état de fonctionnement pendant le processus de chromatographie et commander en temps réel le début et la fin des différentes étapes du processus de chromatographie selon l'état de fonctionnement déterminé.

16. Système à contre-courant périodique de chromatographie comprenant au moins deux colonnes (39, 47, 59 ; 107, 109, 111, 113), le système à courant périodique de chromatographie est configuré pour :

- détecter un signal d'alimentation (201) représentatif de la composition d'un matériau d'alimentation présenté à l'entrée des colonnes ;
- détecter des signaux d'effluent (203, 205, 207, 209) représentatifs de la composition de l'effluent en provenance de chaque colonne dans le système ;
- déterminer l'état de fonctionnement de chaque colonne de chromatographie selon l'une quelconque des revendications 1-11 ; et
- commander l'alimentation vers et entre les colonnes en fonction de l'état de fonctionnement déterminé.

17. Système à contre-courant périodique de chromatographie selon la revendication 16, configuré en outre pour :

a) déterminer en continu l'état de fonctionnement pendant le processus de chromatographie et commander en temps réel l'alimentation vers et entre les colonnes en fonction de l'état de fonctionnement déterminé ;
b) commander les débits des pompes d'alimentation et des pompes tampons (31, 37 ; 101, 103, 105) en fonction de l'état de fonctionnement déterminé ; et/ou
c) compenser les différences éventuelles entre les propriétés et/ou les débits dans les différentes colonnes en ajustant la durée et la position des différentes colonnes dans la zone de chargement en fonction de l'état de fonctionnement déterminé.

Fig. 1

Fig. 2

= T-valve

Fig. 3

35

Fig. 4a

49

Fig. 4b

51

Fig. 4c

Fig. 5

Fig. 6

EP 3 586 123 B1

165

169

171

167

153

R

155

161

159

163

157

151

# Fig. 7

210

260

270

290

280

240

250   230   220   295

# Fig. 8a

Fig. 8b

Fig. 8c

1 LOOP = 1 Elution; 1 CYCLE = 3 Elutions

**Loading**

Feed → UV Sample Pre → In → (columns 2, 3) → Out → UV Sample Post, UV, Cond, pH Prod/PLW, UV Recirculation Waste

Loading: Column 1 to 2
Column 3: Idle

**Wash 1**

Feed → UV Sample Pre → In → (columns 1, 2, 3) → Out → UV Sample Post, UV, Cond, pH Prod/PLW, UV Recirculation Waste

Loading: Column 2
Washing: Column 1 to 3

**Washes 2 & 3, Elution, Strip**

Feed → UV Sample Pre → In → (columns 1, 2, 3) → Out → UV Sample Post, UV, Cond, pH Prod/PLW, UV Recirculation Waste

Wash 2&3, Elution, Strip: Column 1
Loading: Column 2 to 3

Fig. 9a

EP 3 586 123 B1

Fig. 9b

Pre column UV signal (feed line) — — — Post column UV signal (breakthrough)

Desired breakthrough level (% ΔUV)

Total** UV signal

*Media components and impurities
**Product, media components and impurities

Product UV signal

Background' UV signal

Baseline

UV

Time

Fig. 10

Fig. 11a

Fig. 11b

Fig. 12a

Fig. 12b

Fig. 12c

Fig. 12d

Fig. 13a

Fig. 13b

10 g/L titer, mAb 1 low UV

Wash, Elution Strip

ΔUV

UV pre sample

UV post sample

Fig. 13c

Fig. 13d

Fig. 14a

Fig. 14b

Fig. 14c

Fig. 14d

Fig. 15

EP 3 586 123 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 3291726 A
- WO 2010151214 A1
- US 8649005 B
- US 20140255994 A1

### Non-patent literature cited in the description

- **BISCHOPS, M. ; PENNINGS, M.** Simulated Moving Bed technology in Biopharmaceutical Processing. *Recovery Biological Products,* 2003, vol. XI
- **HEETER, G.A. ; LIAPIS, A.I.** *J. Chrom A,* 1995, vol. 711
- **LACKI, K.M. ; BRYNTESSON, L.M.** Protein A Counter-Current Chromatography for Continuous Antibody Purification. *ACS,* 2004
- **ANDERSON, N.G.** Practical Use of Continuous Processing in Developing and Scaling Up Laboratory Processes. *Org. Process Res. Dev.,* 2001, vol. 5 (6), 613-621
- **LAIRD, T.** Continuous Processes in Small-Scale Manufacture. *Org. Process Res. Dev.,* 2007, vol. 11 (6), 927
- Continuous Processing in Pharmaceutical Manufacturing. **MASAK, J.** Chem. Listy. vol. 109, 475
- **ALLISON, G.** Regulatory and Quality Considerations for Continuous Manufacturing. *Presentation at the International Symposium on Continuous Manufacturing of Pharmaceuticals: Implementation, Technology, & Regulatory (Cambridge, MA),* 2014
- **CHATTERJEE, S.** FDA Perspective on Continuous Manufacturing. *Presentation at the IFPAC Annual Meeting (Baltimore, MD),* 2012
- **HERNANDEZ, R.** Continuous Manufacturing: A Changing Processing Paradigm. *BioPharm International,* 2015, vol. 28 (4), 20-27, 41
- **GODAWAT, R. et al.** End-to-end integrated fully continuous production of recombinant monoclonal antibodies. *J Biotechnol.,* vol. 213, 13-9
- **LI, L. et al.** A single-use, scalable perfusion bioreactor system. *BioProcess Int.,* 2009, vol. 7 (6), 46, , 48, , 50, , 52-54
- **VOISARD, D. et al.** Potential of cell retention techniques for large-scale high-density perfusion culture of suspended mammalian cells. *Biotechnol. Bioeng.,* 2003, vol. 82 (7), 751-765
- **WALTHER, J. et al.** Multi-staged experimental design for efficient process development of a continuous perfusion cell culture. American Chemical Society
- **ANGARITA, M. et al.** Twin-column CaptureSMB: a novel cyclic process for protein A affinity chromatography. *J Chromatogr A.,* vol. 1389, 85-95
- **GODAWAT, R. et al.** Periodic counter-current chromatography - design and operational considerations for integrated and continuous purification of proteins. *Biotechnol. J.,* vol. 7 (12), 1496-1508
- **LACKI, K.M. ; L.M. BRYNTESSON.** Protein A periodic counter-current chromatography for continuous antibody purification. American Chemical Society, 2004
- **NG, C.K.S. et al.** Design of high productivity sequential multi-column chromatography for antibody capture. *Food Bioprod. Process.,* vol. 92 (2), 233-241
- **TRILISKY, E. et al.** Capacity assessment: DBC is not the whole story - implications for resin lifetime and sequential multicolumn chromatography. American Chemical Society
- **BLOM, H. et al.** The use of dynamic control in periodic counter current chromatography. American Chemical Society
- **POLLOCK, J. et al.** Optimising the design and operation of semi-continuous affinity chromatography for clinical and commercial manufacture. *J Chromatogr A.,* vol. 1284, 17-27
- **WARIKOO, V. et al.** Integrated continuous production of recombinant therapeutic proteins. *Biotechnol Bioeng.,* vol. 109 (12), 3018-29
- **COOPER, R.S. ; D.A. LIBERMAN.** Fixed-bed adsorption kinetics with pore diffusion control. *Ind. Eng. Chem., Fundam.,* 1970, vol. 9 (4), 620-3